Europäisches Patentamt

European Patent Office

Office européen des brevets

(19)

(11) **EP 0 643 292 B1**

(12) **EUROPÄISCHE PATENTSCHRIFT**

(45) Veröffentlichungstag und Bekanntmachung des Hinweises auf die Patenterteilung:
**11.06.1997  Patentblatt 1997/24**

(51) Int Cl.6: **G01N 1/10**, G01N 33/04, A01J 7/00

(21) Anmeldenummer: **94113638.4**

(22) Anmeldetag: **31.08.1994**

(54) **Verfahren und Vorrichtung zum Entnehmen einer für die abgemolkene Milchgesamtmenge einer Kuh repräsentativen Analysenprobe**

Method and device for taking a sample representative of the milk yield of a cow

Méthode et dispositif de prise d'un échantillon représentatif de la production de lait d'une vache

(84) Benannte Vertragsstaaten:
**AT BE CH DE DK ES FR GB IE IT LI NL PT SE**

(30) Priorität: **14.09.1993  DE 4331203**

(43) Veröffentlichungstag der Anmeldung:
**15.03.1995  Patentblatt 1995/11**

(73) Patentinhaber: **BIO-MELKTECHNIK HOEFELMAYR & Co.
CH-9052 Niederteufen (CH)**

(72) Erfinder: **Hoefelmayr, Tilman, Dr.
CH-9052 Niederteufen (CH)**

(74) Vertreter: **Grünecker, Kinkeldey, Stockmair & Schwanhäusser Anwaltssozietät
Maximilianstrasse 58
80538 München (DE)**

(56) Entgegenhaltungen:
EP-A- 0 098 966          WO-A-82/04127
DE-A- 3 210 465          DE-A- 3 216 537
DE-A- 3 424 179          FR-A- 2 621 390
US-A- 3 308 669          US-A- 4 292 994

**Beschreibung**

Die Erfindung betrifft ein Verfahren zum Entnehmen einer für die abgemolkene Milchgesamtmenge einer Kuh repräsentativen Analysenprobe, wobei während des Abmelkvorgangs der Milch in Abhängigkeit von dem Milchfluß, d. h. der pro Zeit abgemolkenen Milchmenge, mengenproportionale Probenteilmengen entnommen werden, sowie eine Milchprobenentnahmevorrichtung mit einem in einer Melkleitung angeordneten Milchflußmesser sowie einer Prozessoreinheit zur Steuerung einer mit einem Analyseprobenbehälter verbundenen Milchprobenentnahmevorrichtung, die mit der Melkleitung in Verbindung steht.

Die Güte einer Milch und der dafür gezahlte Preis pro kg richten sich weitgehend nach deren Inhaltsstoffen, insbesondere nach dem prozentualen Fettgehalt der Milch. Die Bestimmung des Fettgehalts ist jedoch erheblich erschwert, wenn die Milch über eine Zeit gestanden hat, da sich hierbei der Fettanteil bevorzugt auf der Oberfläche der Milch absetzt. Es bestehen deshalb sehr strenge Vorschriften, wie bei der Entnahme einer sog. repräsentativen Analyseprobe, die normalerweise nicht mehr als 50 ml umfassen soll, vorzugehen ist. Dieses Verfahren ist reichlich umständlich, arbeitsintensiv und auch sehr zeitaufwendig.

Aus der DE 35 28 827 ist bereits eine Probenentnahmevorrichtung bekannt, bei der die Milchproben bei der Überführung der vom Bauern gelieferten Milch in einen Milchtankzug entnommen werden. Hierbei wird aufgrund der von den Vortagen bekannten angelieferten Milchmenge eine sog. erwartete Milchmenge angenommen, und diese Milchmenge wird jeweils durch eine gleiche, vorbestimmte Anzahl von Entnahmeimpulsen geteilt. Hierdurch werden jedem Entnahmeimpuls eine gleiche Milchmenge zugemessen, die multipliziert mit der Anzahl der Impulse die erwartete Gesamtmilchmenge ergibt. In Abhängigkeit von der erwarteten Gesamtmenge können natürlich unterschiedliche Volumenmengen jedem Impuls zugeteilt werden. Bei der Milchförderung wird sodann der Milchvolumenmesser derart eingestellt, daß er nach der errechneten Teilvolumenmenge jeweils einen Impuls abgibt. Mit jedem Impuls wird aus der Milch eine vorbestimmte, jeweils gleiche Probenteilmenge entnommen. Auf diese Weise wird die insgesamt entnommene Probenmenge, die das Produkt aus der festen Anzahl von Impulsen und der festen Probenteilmenge ist, praktisch konstant gehalten.

Aus der WO 82/04127 war ebenfalls bereits eine Vorrichtung zur Entnahme einer vorbestimmten Probenmenge beim Überführen einer größeren vorbekannten Milchmenge von einem Behälter in einen anderen Behälter bekannt. Hierbei wird die Milch in jeweils gleichbleibenden kleineren Milchmengen von etwa 10 l gefördert und es wird eine dieser Fördermenge mengenproportionale Teilprobenmenge über ein Probenentnahmeventil entnommen, wobei die Größe der jeweiligen Öffnung des Ventils, die Öffnungszeit, sowie die Öffnungsfrequenz entsprechend der vorbekannten zu fördernden Milchgesamtmenge und der konstanten Fördermenge pro Zeit vorab eingestellt werden.

Bei dem Gebrauchsmuster G 85 02 259.4 handelt es sich wiederum um eine Milchfördereinrichtung für Tankwagen oder innerbetrieblichen Milchtransport in Molkereien, wo in ähnlicher Weise die Zahl der zu entnehmenden konstanten Probenteilmengen festgelegt wird, indem die Menge jeder zu entnehmenden Probenteilmenge im Verhältnis zu der gesamt zu entnehmenden Probenmenge und die Zeit, während der diese Probenteilmenge entnommen wird, festgelegt werden. Da sich bei der Überführung der Gesamtmilchmenge jedoch Unterschiede im gesamten Milchfluß ergeben können, wurde vorgeschlagen, den Faktor aus dem Verhältnis von der gesamt zu überführenden Milchmenge zu dem jeweils gemessenen Milchfluß zu berücksichtigen, um die Pausenzeiten zwischen zwei Teilprobenentnahmen entsprechend in Abhängigkeit von dem jeweiligen Milchfluß zu verändern. Die zu erwartenden und hierdurch zu berücksichtigenden Milchflußänderungen sind jedoch verhältnismäßig klein.

Aus der DE 32 10 465 ist die mengenproportionale Entnahme von Milch aus dem während des Melkvorganges unmittelbar auftretenden Milchfluß bekannt. Hierbei wird in Verbindung mit einem Milchflußmesser, der von der abgemolkenen Milch durchströmt wird, jeweils eine Teilmenge mit Hilfe einer peristaltischen Pumpe abgezweigt, indem die Laufgeschwindigkeit der peristaltischen Pumpe in Abhängigkeit von dem jeweils gemessenen Milchfluß bzw. der in dem Milchflußmesser gemessenen Füllstandshöhe gesteuert wird. Eine solche Anordnung ist jedoch nur für einen bestimmten Bereich von auftretenden Milchflüssen benutzbar, wobei insbesondere die peristaltische Pumpe bei höheren Milchflüssen keine genaue Probenentnahme mehr gewährleistet. Darüber hinaus weist eine peristaltische Pumpe gerade bei höheren Umlaufgeschwindigkeiten nur eine äußerst beschränkte Lebensdauer und einen relativ hohen Energiebedarf auf, der sich noch dadurch erhöht, daß die Pumpe zwischen Vakuum und Atmosphärendruck arbeiten muß.

Die gleichen Nachteile hat die aus der DE 32 16 537 bekannt-gewordene Anordnung, bei der zur Milchflußmessung eine erste peristaltische Pumpe verwandt wird, während zur Probenentnahme eine zweite peristaltische Pumpe dient. Zu der Verdoppelung des Energieverbrauchs kommt zusätzlich noch ein erhebliches Gewicht der Gesamtanordnung, die eine solche Anordnung für die Verwendung in einem transportablen Handgerät ausschließen. Zudem ergibt sich bei einer solchen Anordnung eine verhältnismäßig hohe, sog. Verschleppungsgefahr, durch Milchreste, die von der vorgehenden Probe in der Meßvorrichtung zurückbleiben und in die nächstfolgende Meßprobe gelangen.

Wie bereits erläutert, werden sog. repräsentative Proben hauptsächlich im Hinblick auf eine genaue Feststellung des Fettgehaltes der abgemolkenen Milchmenge durchgeführt. Da das Fett jedoch beim Stehen der Milch dazu neigt, sich verhältnismäßig schnell von den übrigen Milchbestandteilen abzusetzen, bedarf es einer umständlichen längeren genauen Vorgehensweise, um aus einer bereits abgestandenen Milch eine repräsentative Probe zu entnehmen.

Ein solches Verfahren könnte natürlich entfallen, wenn es möglich wäre, eine derartige repräsentative Probe unmittelbar beim Abmelken einer Kuh, d.h. wenn die Milch noch nicht abgestanden ist, zu entnehmen. Ein solches Verfahren wäre auch wünschenswert im Hinblick darauf, daß jede Kuh gesondert überwacht und der Fettgehalt der Milch getrennt bestimmt werden könnte. Die Schwierigkeit eines solchen Verfahrens liegt jedoch darin, daß trotz erheblich schwankender Gesamtmilchmengen pro Kuh und trotz starker von Kuh zu Kuh und zeitlich während jedes Melkvorgangs differierender Milchflüsse jeweils nur eine kleine Analysenprobenflasche von weniger als 50 ml Inhalt zur Verfügung steht, in die eine repräsentative Probe direkt eingefüllt werden soll. So differieren die Erwartungswerte der Milchgesamtmenge einer Kuh bereits zwischen etwa 5 und 30 kg, also in einem Verhältnis 1:6, während die Milchflüsse, die während eines Melkvorganges auftreten können, zwischen 0,1 und 12 kg pro Minute, also in einem Verhältnis 1:120 schwanken können. Nimmt man beide Einflußgrößen der Gesamtmilchmenge und des Milchflusses zusammen, so ergibt sich eine mögliche Spannbreite von möglichen zu erwartenden Änderungen in einem Verhältnis von 1:720. Berücksichtigt man gleichzeitig die Vorgabe, daß die Menge der Analysenprobe lediglich zwischen 20 und 40 ml, d.h. höchstens in einem Verhältnis 1:2 schwanken kann, so ergibt sich immer noch eine abzudeckende Variationsbreite der zu berücksichtigenden Haupteinflußfaktoren von 1:360.

Mit den bisher zu erwartenden Pumpen läßt sich dieses Problem nicht lösen. So läßt sich eine Peristaltik-Pumpe, die vorbestimmte Volumina durch Abquetschen eines Schlauches fördert, nicht über einen Bereich von 1:360 steuern, da ihr maximaler Drehzahlbereich lediglich 1:100 beträgt und hierzu eine aufwendige Gleichstromsteuerung notwendig ist. Insbesondere ist dabei die Steuerung des hierzu benötigten Gleichstrommotors im unteren Drehzahlbereich sehr problematisch. Abgesehen davon würde sich eine derartige Peristaltikpumpe nur schwerlich für eine an der Kuh zu verwendende, tragbare Meßeinrichtung eignen, da sie neben einem verhältnismäßig hohen Energieverbrauch ein hohes Gewicht und auch ein großes Bauvolumen benötigt. Zudem ist die Lebensdauer der notwendigen Schläuche klein, da sich die Elastizität dieser Schläuche mit der Zeit ändert, wodurch sich gleichzeitig das geförderte Volumen ändert.

In gleicher Weise vermag eine mit Membran arbeitende Elektromagnetpumpe den geforderten Bereich von 1:360 nicht abzudecken. Außerdem sind in Verbindung mit einer solchen Pumpe Ventile notwendig, durch die sich jeweils das Problem einer ausreichenden Reinigung stellt, da sie im Betrieb bevorzugt dazu neigen, daß sich Milch festsetzt, d.h. daß eine erhöhte Gefahr der Verkäsung entsteht. Schließlich haben aber auch solche Elektromagnetpumpen einen verhältnismäßig hohen Stromverbrauch.

Die vorliegende Erfindung strebt deshalb ein Verfahren sowie eine Vorrichtung an, mit der für jede Kuh getrennt während des Melkens eine repräsentative Analyseprobe von weniger als 50 ml gezogen werden kann.

Dies wird bei einem Verfahren der eingangs erwähnten Art dadurch erreicht, daß unter Verwendung eines in Zyklen gesteuerten Ventils, wobei jeder Zyklus eine Ventiloffenzeit und eine Ventilgeschlossenzeit umfaßt, zur Entnahme lediglich einer unter einer vorbestimmten Maximalmenge von 50 ml gehaltenen Analyseprobe aus dem aus der Erfahrung gewonnenen Erwartungswert der abzumelkenden Milchgesamtmenge der betreffenden Kuh eine Zykluszeit und eine Ventiloffenzeit bestimmt werden, wobei Zykluszeit und Ventiloffenzeit jeweils derart gewählt werden, daß sie innerhalb vorbestimmter Wertebereiche liegen, daß die Ventiloffenzeit oder die Zykluszeit in Abhängigkeit von dem jeweils gemessenen Milchfluß gesteuert werden, und daß zur Vermeidung von außerhalb der Wertebereiche liegenden Werten der Ventiloffenzeit oder der Zykluszeit infolge der Veränderung des Milchflusses die Ventiloffenzeit und die Zykluszeit in gleichen Verhältnissen zu innerhalb der Wertebereiche liegenden Werten verändert werden.

Mit einem solchen Verfahren lassen sich repräsentative Analysenproben in einem großen Schwankungsbereich der zu erwartenden Gesamtmilchmenge zwischen 5 und 30 kg sowie eines möglichen Milchflusses zwischen 0,1 und 12 kg pro Minute entnehmen.

Zur Erleichterung des Verfahrens werden die abgespaltenen Probenteilmengen unter dem gleichen Druck gehalten wie der Milchfluß selbst.

Zur Vereinfachung der Steuerung wird vorzugsweise zur Erzeugung eines im wesentlichen von dem Milchfluß unabhängigen Abspaltflusses durch das Ventil die Milchstauhöhe über dem Ventil konstant gehalten.

Demgegenüber kann der Meßbereich noch erweitert werden, wenn das Verfahren derart durchgeführt wird, daß über dem Ventil eine jeweils von dem Milchfluß abhängige Milchstauhöhe zur Änderung des Abspaltflusses durch das Ventil in Abhängigkeit vom Milchfluß erzeugt wird.

Der Wertebereich für die Zykluszeit, in dem diese veränderbar ist, wird an der unteren Grenze praktisch nur durch die Steuerbarkeit der reproduzierbaren Milchabspaltmenge des Ventils und am oberen Ende durch die Zahl der zu entnehmenden Proben bei kleinen Milchflüssen zur Erzielung einer repräsentativen Probe bestimmt und liegt vorzugsweise zwischen 0,5 und 30 Sekunden (120 bis 2 Zyklen pro Minute). Bevorzugt liegt der Wertebereich jedoch zwischen 2 und 30 Sekunden.

Der Wertebereich für die Ventiloffenzeit bestimmt sich bei niedrigen Offenzeiten im wesentlichen durch die Trägheit des Ventils und bei den oberen Offenzeiten daraus, bis zu welchen Offenzeiten bei konstantem Milchfluß ein konstanter Abspaltfluß erzielbar ist. Der Wertebereich liegt hierbei zwischen 0,05 und 1,2 Sekunden bei hohen Milchflüssen bzw.

vorzugsweise zwischen 0,1 und 0,8 Sekunden und kann sich bei sehr kleinen Milchflüssen auf den Bereich von 0,1 bis 0,25 Sekunden reduzieren.

Wie bekannt ist, nimmt der Fettgehalt der abgemolkenen Milch zum Ende des Melkvorgangs hin zu. Dadurch ändert sich auch die Viskosität und das Fließverhalten der Milch leicht. In diesem Zusammenhang kann auch der Einfluß von Kapillarkräften von Bedeutung werden. Aus diesem Grunde kann es zur Feinkalibrierung des in der Analyseprobe enthaltenen Fettgehalts mit zunehmender Verringerung des Milchflusses gegen Melkende hin zweckmäßig sein, die Ventiloffenzeit zunehmend etwas zu vergrößern oder zu verkleinern.

Vorzugsweise wird das Verfahren so durchgeführt, daß während jeder Schließung des Ventils ein Teil der durch das Ventil geflossenen Milch zurückgepumpt wird und vorteilhafterweise wird das Verfahren weiter auch derart durchgeführt, daß bei jedem Öffnen des Ventils eine das Anlaufen des Milchflusses beschleunigende Ansaugkraft auf die Milch ausgeübt wird.

Die Erfindung betrifft auch eine Milchprobenentnahmevorrichtung der eingangs erwähnten Art, die sich dadurch auszeichnet, daß die Milchprobenenentnahmevorrichtung eine elektrisch steuerbare Magnetspule umfaßt, mit der ein Verschlußkörper in eine erste, eine Durchflußöffnung für den Probenabspaltstrom versperrende und eine zweite, diese Öffnung freigebende Stellung bewegbar ist, daß der Verschlußkörper mit einer steuerbaren Zykluszeit zwischen der ersten und zweiten Stellung hin- und herbewegbar ist, und daß die Zykluszeit und die Zeit (Ventiloffenzeit), in der sich der Verschlußkörper in der die Durchflußöffnung freigebenden zweiten Stellung befindet in Abhängigkeit von der zu erwartenden Gesamtmilchmenge jeweils derart wählbar sind, daß sie innerhalb vorbestimmter Wertebereiche liegen und wobei die Ventiloffenzeit oder die Zykluszeit in Abhängigkeit von dem jeweils gemessenen Milchfluß über die Prozessoreinheit steuerbar ist, und daß zur Vermeidung von außerhalb der Wertebereiche liegenden Werte der Ventiloffenzeit oder der Zykluszeit infolge der Veränderung des Milchflusses die Ventiloffenzeit und die Zykluszeit in gleichen Verhältnissen zu innerhalb der Wertebereiche liegenden Werten verändert werden.

Vorzugsweise besteht der Verschlußkörper aus einem Permanentmagneten bzw. ferromagnetischem Material, und in der Nähe der Durchflußöffnung ist ein den Verschlußkörper in seiner ersten Stellung haltender Körper aus einem ferromagnetischem Material bzw. aus einem Permanentmagneten angeordnet. Diese Ausgestaltung ermöglicht einen energiesparenden Betrieb, da jeweils lediglich ein Umschaltimpuls von kurzer Dauer notwendig ist, um den Verschlußkörper von einer ersten in die zweite Stellung und umgekehrt zu bewegen. Dies kann jeweils durch kurze Impulse von etwa einer Impulsdauer von 10 bis 100 ms erfolgen, die jeweils aufeinanderfolgend einen Strom von entgegengesetzter Stromrichtung führen. Die Umschaltung in der Stromrichtung kann etwa dadurch erfolgen, daß lediglich die Spannung an den Enden der Magnetspule umgepolt wird, was mit Hilfe elektronischer Steuerungen erfolgen kann.

In der Praxis hat es sich als besonders zweckmäßig erwiesen, daß der Verschlußkörper aus einem zylindrischen Körper besteht, der in seitlichen Führungen geführt ist. Der Körper bzw. die Führungen soll zweckmäßigerweise aus einem solchen Material bestehen oder mit einer solchen Materialauflage versehen sein, daß sich eine möglichst niedrige Reibung zwischen den Führungen und dem Körper ergibt.

Zweckmäßigerweise bildet ein die Durchflußöffnung für den Probenentnahmestrom bestimmendes Rohr den ferromagnetischen Körper bzw. den aus einem Permanentmagneten bestehenden Körper.

Zur optimalen Abdichtung und auch zur Herabsetzung der Betriebsgeräusche und zur Verlängerung der Lebenszeit ist der Verschlußkörper und/oder das dem Verschlußkörper zugewandte Ende der Durchflußöffnung mit einer Lage aus einem Dämpfungsmaterial versehen. Ein solches Dämpfungsmaterial kann etwa in Form einer Platte aus z.B. Silikon oder Polyurethan oder aber auch in Form eines Federstahldrahtes, der mit Silikon umhüllt ist, bestehen.

Zur Erhöhung der Betriebssicherheit und Betriebsgenauigkeit wird die Magnetspule in Höhe der zweiten Stellung des Verschlußkörpers angeordnet.

Um ein einwandfreies Ausschleusen der für die Probenentnahme abgespaltenen Milchmenge unter Gleichdruck zu ermöglichen, wird eine besondere Leitung vorgesehen, die einerseits mit dem Melkvakuum in Verbindung steht und andererseits bis zum Ende der Milchprobenentnahmevorrichtung geführt ist. Diese entläßt aus der Analyseflasche die Luft, die von der Milchfüllung verdrängt wird. Auf diese Weise wird sichergestellt, daß sowohl über dem Milchfluß, aus dem eine Milchprobe abgespalten werden soll, wie auch an dem Auslaßende der Milchprobenentnahmevorrichtung der gleiche Druck, nämlich im vorliegenden Falle Melkvakuum, herrscht.

Um die Verschleppungsgefahr durch die Milchprobenentnahmeeinrichtung möglichst gering zu halten, werden einerseits die für den Milchdurchfluß vorgesehenen Räume möglichst klein, jedoch andererseits jedoch auch so ausgebildet sein, daß Milch gut abfließt und sich die Milchprobenentnahmeeinrichtung auch möglichst gut reinigen läßt. Als vorteilhaft hat sich eine Anordnung herausgestellt, bei der der zylinderförmige Verschlußkörper entlang einer im wesentlichen zylinderförmigen Führungsbahn beweglich ist, in deren dem Verschlußkörper zugewandten Fläche in Längsrichtung des Verschlußkörpers verlaufende Ausnehmungen vorgesehen sind, die in der zweiten Stellung des schlußkörpers mit der Durchflußöffnung und dem dem probenbehälter zugewandten Ende der Milchprobenentnahmeeinrichtung in Verbindung stehen.

Zum Erreichen einer einwandfreien Funktion des Ventils, insbesondere bei niedrigen Ventilöffnungszeiten, wie auch zur Verhinderung der Verstopfung, insbesondere der Verkäsung der Durchflußöffnung, ist es zweckmäßig, die Ausgestaltung derart zu treffen, daß die Verbindung zwischen der Durchflußöffnung und den Ausnehmungen zum Ableiten der Milch bei einer Verstellbewegung des Verschlußkörpers über eine vorbestimmte Strecke D vor Erreichen der Verschließstellung der Durchflußöffnung bzw. während der Öffnungsbewegung des Verschlußkörpers im wesentlichen unterbrochen ist.

Gemäß einer bevorzugten Ausführungsform wird die Milchprobenentnahmeeinrichtung unmittelbar mit einem Raum verbunden, in dem sich eine dem jeweiligen Milchfluß entsprechende Milchstauhöhe einstellt. Dies ist vorzugsweise ein entsprechender Raum des verwandten Milchflußmessers. Zur Messung der Stauhöhe können in diesem Raum auch noch zusätzliche Einrichtungen vorgesehen sein, wie etwa in der Höhe im Abstand voneinander angeordneten Meßsonden.

Ein geringerer Rechen- und Steueraufwand wird dann benötigt, wenn entsprechend einer weiteren Ausführungsform die probenentnahmevorrichtung in einen Raum mündet, in dem der Milchfluß jeweils auf einer vorbestimmten Stauhöhe gehalten wird. Dies kann beispielsweise durch eine entsprechende Verbindung mit einem entsprechend ausgebildeten Milchflußmesser, und zwar in einem sog. Sumpfbereich erreicht werden.

Während bei den vorhergehenden Ausführungsformen eine Abspaltung entsprechend dem hydrostatischen Druck erfolgte, kann gemäß einer anderen Ausführungsform eine Abspaltung auch dadurch erreicht werden, daß die Durchflußöffnung der probenentnahmevorrichtung mit einem in eine Milchtransportleitung hinein vorstehenden Probenentnahmerohr verbunden ist. In diesem Fall erfolgt eine dynamische Probenentnahme.

Es hat sich in diesem Falle als zweckmäßig erwiesen, das Probenentnahmerohr mit der Längsachse seiner Einlaßöffnung so anzuordnen, daß diese etwa im Abstand von 1/3 des Durchmessers der Milchtransportleitung von der Innenwand der Milchtransportleitung angeordnet ist.

Gemäß einer bevorzugten Ausführungsform bei der dynamischen Probenentnahme verzweigt sich das Probenentnahmerohr in eine erste, die Durchlaßöffnung umfassende und mit einer Ableitung in den Analysenprobenbehälter verbundenen Leitung und eine zweite Leitung, die mit dem Milchabfluß verbunden ist, wobei der Verschlußkörper derart bewegbar ist, daß er in seiner ersten Stellung die Durchlaßöffnung verschließt und die zweite Leitung freigibt und in seiner zweiten Stellung die Durchlaßöffnung und die erste Leitung freigibt und die zweite Leitung verschließt.

Im folgenden soll die Erfindung näher anhand von in der Zeichnung dargestellten Ausführungsform erläutert werden. In der Zeichnung zeigen:

Fig.1     eine schematische Darstellung einer Melkanordnung, bei dem das erfindungsgemäße Verfahren und die entnahmevorrichtung einsetzbar sind;

Fig.2     eine schematische Darstellung eines Milchflußmessers im Schnitt sowie einer Milchprobenentnahmeeinrichtung gemäß der Erfindung;

Fig.3     eine schematische Darstellung eines Milchflußmessers im Schnitt mit einer Milchprobenentnahmeeinrichtung gemäß der Erfindung in einer anderen Kombination;

Fig.4     eine schematische Darstellung eines Milchflußmessers im Schnitt, mit dem eine Milchprobenentnahmeeinrichtung gemäß der Erfindung in anderer Kombination verbunden ist;

Fig.5     eine schematische Darstellung eines Milchflußmessers im Schnitt in Kombination mit einer weiteren Ausführungsform einer schematisch dargestellten erfindungsgemäßen Milchprobenentnahmeeinrichtung;

Fig.6     ein Längsschnitt durch eine Ausführungsform einer erfindungsgemäßen Milchprobenentnahmeeinrichtung;

Fig.7     eine Schnittansicht einer erfindungsgemäßen Milchprobenentnahmeeinrichtung ähnlich der in Fig. 6 gezeigten Ausführungsform, wobei lediglich im anderen Teil zwei unterschiedliche Befestigungen für eine Analyseprobenflasche dargestellt sind;

Fig.8     eine Schnittansicht einer weiteren Ausführungsform einer gemäß der Erfindung ausgebildeten Milchprobenentnahmeeinrichtung;

Fig.9     ein Schnitt entlang der Linie IX-IX durch die in Fig. 8 gezeigte Ausführungsform; und

Fig.10    eine Milchprobenentnahmeeinrichtung im Schnitt, wie sie in Fig. 4 gezeigt ist.

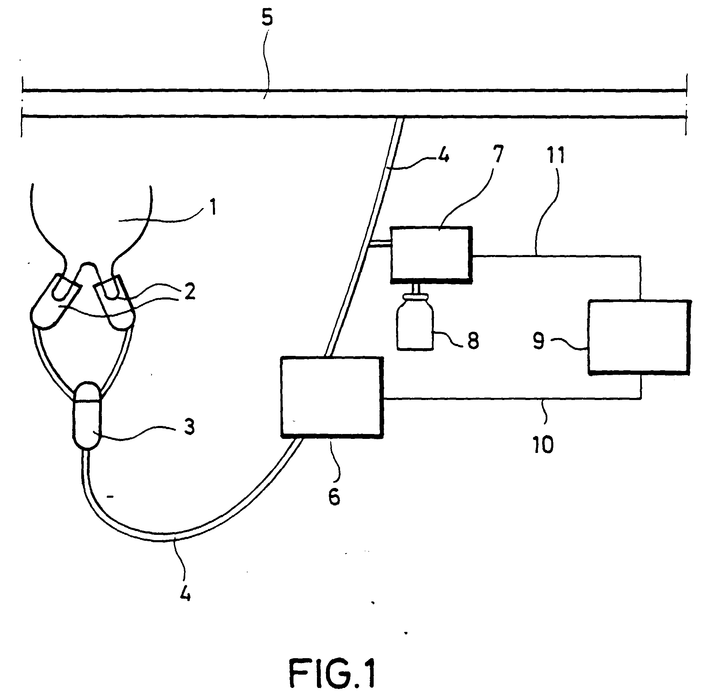

In Fig. 1 ist schematisch das Euter 1 einer Kuh dargestellt, an deren Zitzen Melkbecher 2 angesetzt sind. Die mit diesen Melkbechern abgemolkene Milch wird in einem sog. Milchsammelstück 3 in eine einzige Transportleitung 4 zusammengeführt, die die abgemolkene Milch in eine z.B. über Kopf geführte, unter Melkvakuum stehende Gesamttransportleitung 5 überführt. In dem langen Melkschlauch 4 ist ein Milchflußmesser 6 angeordnet. Weiterhin ist eine mit dem langen Melkschlauch 4, oder auch ggf. mit dem Milchflußmesser 6 verbundene Milchprobenentnahmeeinrichtung 7 dargestellt, die die entnommene Probe in ein Analyseprobenfläschchen 8 sammelt. Ein Prozessor 9, in den bestimmte Vorgabewerte eingebbar sind, empfängt von dem Milchflußmesser 6 über die Leitung 10 Signale entsprechend dem Milchfluß bzw. entsprechend der Milchstauhöhe in dem Milchflußmesser, berechnet ggf. geflossene Milchvolumen oder Änderung des Milchflusses pro Zeit sowie Ventiloffenzeiten und Zykluszeiten, und steuert über die Leitung 11 die Milchprobenentnahmeeinrichtung 7.

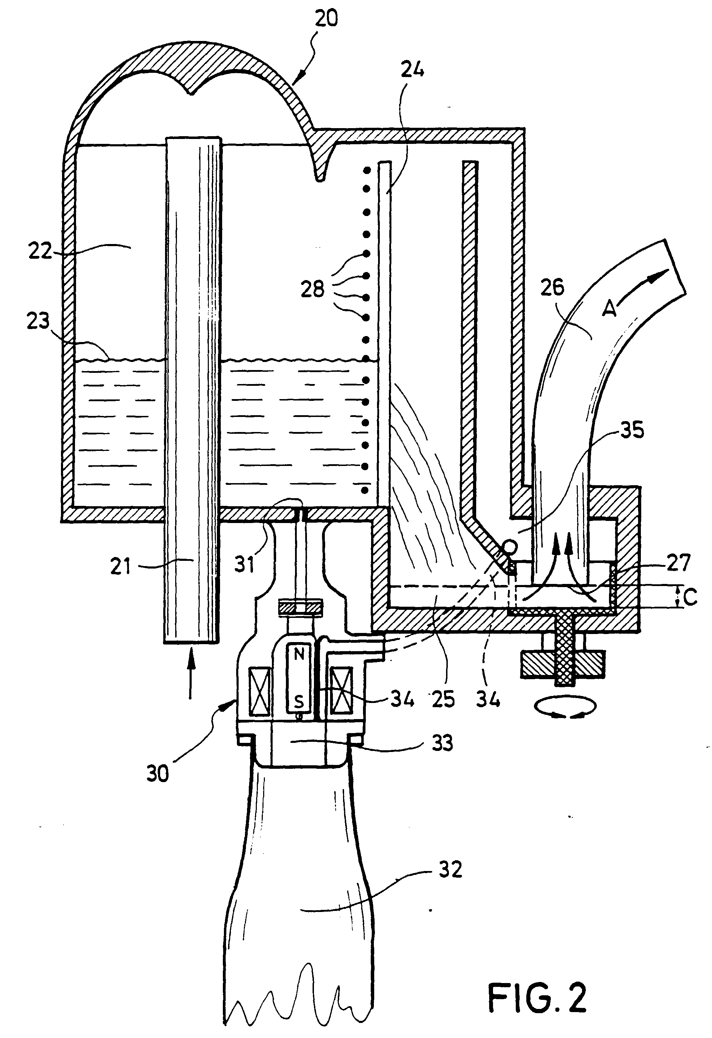

In Fig. 2 ist ein allgemein mit 20 dargestellter Milchflußmesser bezeichnet. Über ein Rohr 21, das Teil des langen Milchschlauches 4 sein kann, gelangt die abgemolkene Milch unter dem anliegenden Melkvakuum aufwärts in den Meß- und Sammelraum 22. Entsprechend dem jeweiligen Milchfluß staut sich die Milch hierin bis zu einer Stauhöhe 23. Die in dem Raum 22 gestaute Milch fließt über einen Meßschlitz 24 aus dem Meß- und Sammelraum ab in einen sog. Sumpf 25. In diesen Sumpf 25 steht von oben her eine Milchabführleitung 26, die gleichfalls Teil des langen Milchschlauches sein kann, vor. Über die Abführleitung 26 wird aufgrund des hierin wirksamen Melkvakuums die Milch in dem Sumpf 25 bis zu der Höhe des unteren Endes 27 der Abführleitung abgesaugt. Die Milch hat in diesem Sumpfteil 25 deshalb jeweils eine konstante Höhe C.

In der Meß- und Sammelkammer 22 sind vor dem Meßschlitz 24 jeweils in der Höhe im Abstand voneinander mehrere Stauhöhensensoren 28 angeordnet, mit denen die Stauhöhe der Milch in dieser Kammer festgestellt werden kann. Für die kontinuierliche oder zyklische Abtastung dieser Stauhöhensensoren 28 kann eine eigene, nicht gezeigte elektrische Abtasteinrichtung in dem Milchflußmesser 20 selbst vorgesehen sein, über den sodann ein einziges Signal entsprechend der Stauhöhe an den Prozessor 9 übermittelt wird. Eine entsprechende Abtasteinrichtung für die Stauhöhensensoren kann aber auch in dem Prozessor 9 vorgesehen sein, der die einzelnen Stauhöhensensoren über die Leitung 10 abtastet und in dem Prozessor selbst ein entsprechendes Stauhöhensignal bildet.

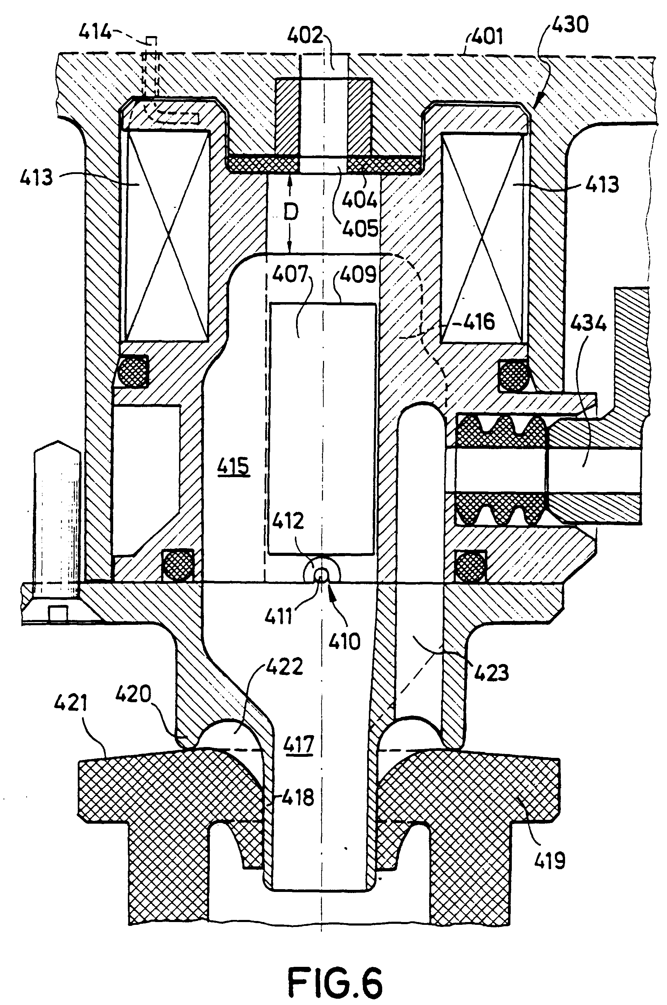

Unterhalb des Milchflußmessers 20 ist eine Milchprobenentnahmeeinrichtung 30 vorgesehen, die über eine kalibrierte Öffnung 31 im Boden der Meß- und Sammelkammer 22 mit dieser verbunden ist. Verschiedene Ausführungsformen dieser Milchprobenentnahmeeinrichtungen 30 sollen später anhand der Figuren 6 bis 10 beschrieben werden.

Am unteren Ende der Milchprobenentnahmeeinrichtung 30 ist eine Analyseprobenflasche 32 befestigt, in die die entnommene Probe abgefüllt wird. Das in diese Analyseprobenflasche 32 hineinragende untere Ende 33 der Milchprobenentnahmeeinrichtung ist über eine mit diesem Ende verbundene Leitung 34 mit dem Raum 35 oberhalb des Milchsamples verbunden. In diesem Teil des Milchflußmessers herrscht Melkvakuum.

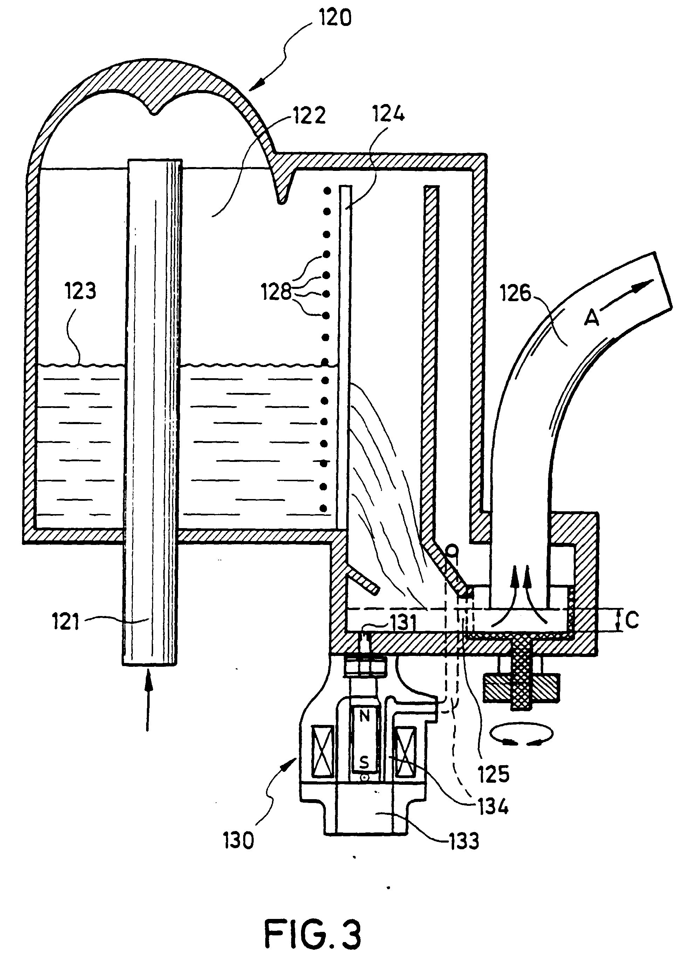

In Fig. 3 ist ein Milchflußmesser sowie eine Milchprobenentnahmeeinrichtung derselben Art wie in Fig. 2 dargestellt, weshalb gleiche Teile mit den gleichen, jedoch um 100 erhöhten Bezugszeichen bezeichnet sind. Die Ausführungsform unterscheidet sich lediglich von der in Fig. 2 gezeigten Ausführungsform dadurch, daß die Zuleitung zu der Milchprobenentnahmeeinrichtung über eine kalibrierte Öffnung 131 im Boden des Gehäuses des Milchflußmessers im Bereich des sog. Sumpfes 125 vorgesehen ist. Im Unterschied zu der in Fig. 2 dargestellten Ausführungsform bleibt die Stauhöhe der Milch im Bereich des Sumpfes auf einer konstanten Höhe C, unabhängig von dem jeweils vorliegenden Gesamtmilchfluß.

Bei den vorstehend beschriebenen Ausführungsformen ist die Milchprobenentnahmeeinrichtung jeweils in Kombination mit einem Milchflußmesser dargestellt. Anstelle des gezeigten Milchflußmessers können natürlich andere Milchflußmesser mit einem anderen Funktionsprinzip, z.B. auch Pfropfen analysierende oder absetzig arbeitende Milchflußmesser eingesetzt werden. Eine Kopplung von Milchflußmesser und Milchprobenentnahmeeinrichtung ist selbstverständlich nicht notwendig, jedoch trägt sie zu einer kompakteren Ausgestaltung des Gesamtgerätes bei. Selbstverständlich könnte die Milchprobenentnahmeeinrichtung auch mit einem gesonderten Raum verbunden sein, durch den die abgemolkene Milch fließt, während sie sich entsprechend dem jeweiligen Milchfluß in einer vorbestimmten Höhe staut oder sie könnte auch mit einem Raum in der Milchtransportleitung verbunden sein, in der die Milch über der Milchprobenentnahmeeinrichtung konstant auf einer vorbestimmten Höhe gehalten wird.

Bei den vorstehenden Ausführungen der Milchprobenentnahmeeinrichtung spielt jeweils der durch die gestaute Milch erzeugte hydrostatische Druck eine Rolle für den Abspaltfluß, der jeweils in die Analyseprobenflasche fließt. Bei den beiden nachfolgend beschriebenen Ausführungsbeispielen findet demgegenüber eine Milchabspaltung mit Hilfe der kinetischen Energie des aus einem Milchsumpf wieder neu beschleunigten Milchstroms statt.

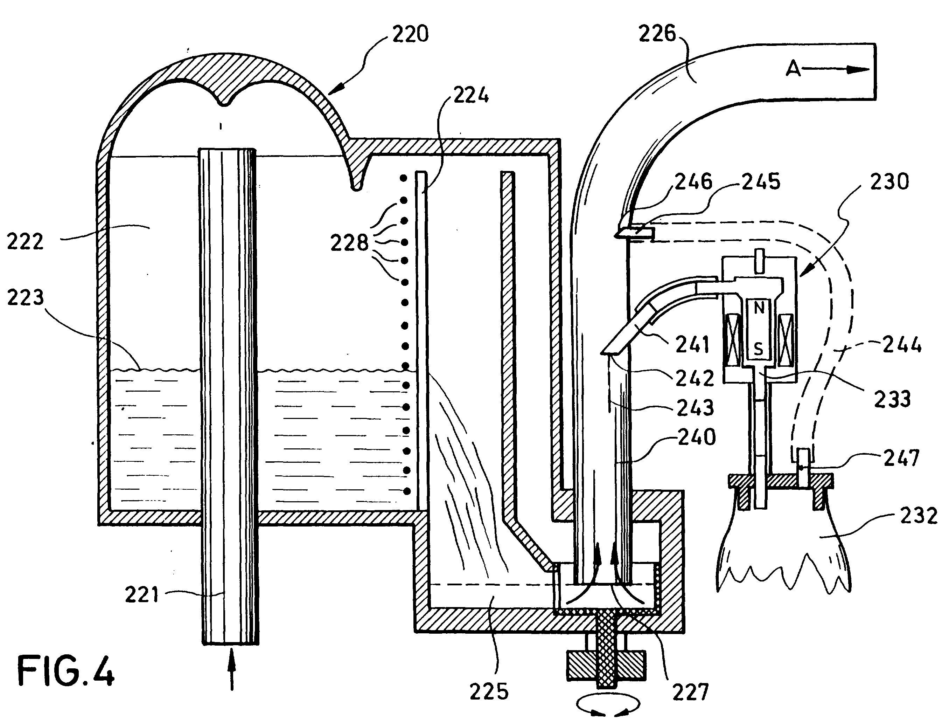

In Fig. 4 sind die gleichen Bauteile mit denselben, jedoch um 200 erhöhten Bezugsziffern wie in Fig. 2 bezeichnet. Aus dem Milchflußmesser 220 wird die Milch über die Abführleitung 226 in Richtung des Pfeiles A abtransportiert. In den senkrecht verlaufenden Teil 240 der Abführleitung 226 steht ein kleines Entnahmeröhrchen 241 hinein vor, dessen nach abwärts gerichtete freie Öffnung 242 im Verhältnis zu der freien Querschnittsfläche der Abführleitung 226 eine um etwa den Faktor 50-100 oder noch geringere freie Querschnittsfläche aufweist. Da die in der Abflußleitung 226 transportierten Milchkolben nicht immer eine gleichförmige Gestalt haben und z.B. an der Innenseite der Leitung 226 durchaus länger ausgebildet sein können als in der Mitte der Rohrleitung, wird, um eine möglichst genaue, mengenmäßig proportionale Abspaltung von Milch aus jedem Kolben zu erreichen, die Mitte 243 der freien Öffnung 242 so angeordnet, daß sie etwa im Abstand von 1/3 des Innendurchmessers der Abflußleitung 226 von deren Innenwand aus gesehen liegt, wenn man sowohl für die Abflußleitung wie auch für das freie Ende 242 etwa kreisförmige Querschnitte annimmt. Würde die Milchprobenentnahmeeinrichtung 230 kontinuierlich geöffnet sein, würde ein vom Milchfluß abhängiger, in guter Näherung reproduzierbarer, aber nicht mengenproportionaler Milchfluß in die Analysenprobenflasche fließen. Um diesen Milchfluß mengenproportional zu machen, müßte er nach einer entsprechenden Kennlinie mit einem Ventil gesteuert werden. Für die Abspaltung einer Probenmenge von weniger als 50 ml müßten jedoch so kleine Röhrchenquerschnitte verwandt werden, daß die Messung zu einen äußerst ungenau würde und sich zum anderen unüberwindbare Reinigungsprobleme einstellen würden.

Damit auch bei diesem System die Abspaltung der Milch unter Gleichdruck erfolgt, ist die Analysenprobenflasche 232 noch über die nur gestrichelt gezeichnete Leitung 244 und über das in das Innere der Abführleitung 226 vorstehende Rohr 245 mit dem Melkvakuum verbunden. Um zu verhindern, daß trotz der dem Milchfluß abgewandten Öffnung 246 des Rohres Milch in die Analysenprobenflasche hineingelangt, ist in der Leitung 244 eine äußerst kleine Bohrung 247 von höchstens 0,5 bis 0,8 mm im Durchmesser vorgesehen. Die Öffnung ist so bemessen, daß ein leichter Luftstrom von der Bohrung 247 durch die Leitung 244 in das Innere der Abführleitung 226 stattfindet, um das Eindringen von Milch in die Leitung 244 von vornherein zu unterbinden. Die Öffnung 247 sollte andererseits aber auch so gering sein, daß sich praktisch kein Vakuumverlust in der Leitung 244 einstellt, da das Innere der Probenanalysenflasche 232 auf dem Melkvakuum gehalten werden soll. Diese Öffnung wird vorzugsweise am Anfang der Leitung 244 in der Nähe des Rohres 245 angebraacht.

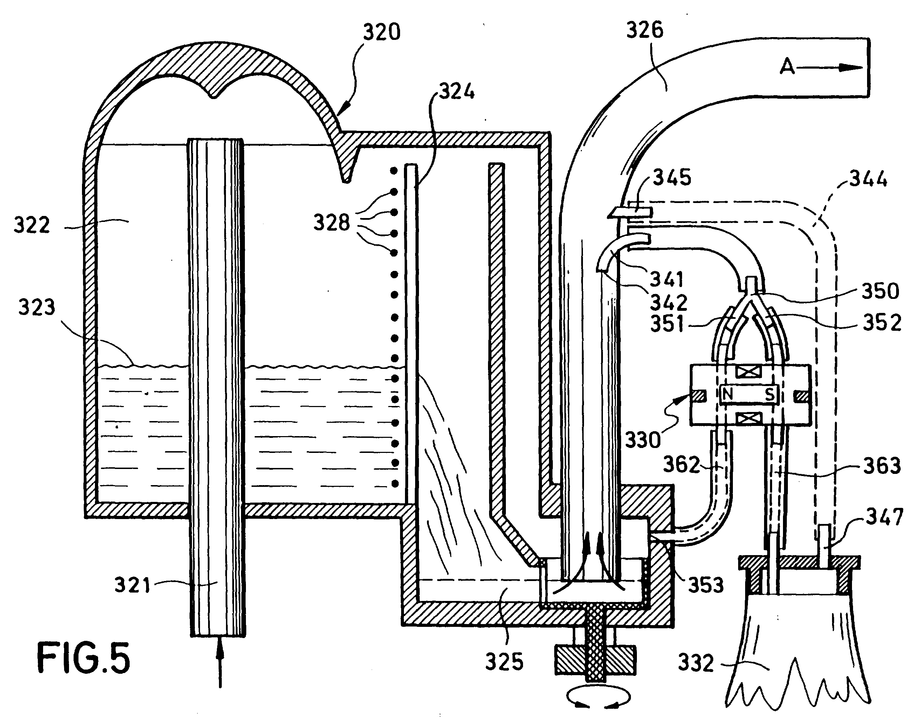

In Fig. 5 ist eine abgewandelte Ausführungsform der Milchprobenentnahmeeinrichtung gegenüber der in Fig. 4 gezeigten Milchprobenentnahmeeinrichtung dargestellt. Die übrigen gleichbleibenden Teile sind mit denselben, jedoch um die Zahl 300 erhöhten Bezugszeichen bezeichnet. Auf diese Teile soll nicht näher nochmals eingegangen werden.

Das in die Abflußleitung 326 vorstehende Rohr 341 ist über eine Verzweigung 350 in zwei Leitungen 351 und 352 aufgespalten, von denen die Leitung 351 über die Milchprobenentnahmeeinrichtung 330 und das Ende 353 der Leitung 351 mit dem Milchsumpf 325 des Milchflußmessers 320 verbunden. Die andere Leitung 352 ist über die Milchprobenentnahmeeinrichtung 330 mit der Analyseprobenflasche 332 verbunden. Die Milchprobenentnahmeeinrichtung 330 umfaßt ein Umschaltventil, wie es im einzelnen näher anhand der Fig. 11 dargestellt ist.

In Fig. 6 ist eine Ausführungsform einer Milchprobenentnahmeeinrichtung dargestellt, wie sie in den Ausführungen der Fig. 2, 3 und 4 verwandt werden kann. So ist die Milchprobenentnahmeeinrichtung 430 derart an die Unterseite des Milchflußmessers 20 ansetzbar, daß die Oberseite 401 gegen die Unterseite des Milchflußgehäuses anliegt und die kalibrierte Öffnung 31 mit der Durchgangsöffnung 402 ausgerichtet ist. Die Durchgangsöffnung 402 wird in ihrem unteren Teil durch einen ringförmigen Körper 403 gebildet, der entweder aus einem Permantmagneten oder aus einem ferromagnetischen Material besteht. Gegen die Unterseite dieses Körpers 403 liegt eine Prallplatte 404 aus einem stoßdämpfenden Material, wie etwa Silikon oder Polyurethan, an. Durch die Dicke dieser Platte kann bei sonst gleichen Bedingungen die Haftkraft des Permanentmagneten genau eingestellt werden. Die Prallplatte weist eine wenigstens gleich große, mit der Durchführung 402 ausgerichtete Öffnung 405 auf. Anstelle der Prallplatte 404 kann auch eine auf die Unterseite des Körpers 403 direkt aufgebrachte Dämpfungsschicht vorgesehen sein. Unterhalb der Prallplatte 404 kann ein gegenüber dem Durchmesser der Öffnung 405 im Durchmesser größerer, im wesentlichen zylindrischer Hohlraum 406 der Höhe D vorgesehen sein. Diese Höhe D ist kürzer als der Gesamtventilhub. Der Durchmesser ist etwas größer als der bewegliche Verschlußteil. Dies ergibt den Kolbeneffekt des Ventils, der weiter unten näher beschrieben wird. In dem Hohlraum ist ein in senkrechter Richtung beweglicher Körper 407 geführt. Der Körper 407 besteht vorzugsweise aus einem kreiszylindrischen Körper, jedoch können auch anders geformte, in dem Hohlraum 406 bewegliche Körper vorgesehen sein, solange dieser lediglich eine Kopffläche 409 aufweist, die sich in einer ersten oberen Stellung dichtend gegen die Prallplatte 404 anlegen kann, um den Durchgang 402 dicht zu verschließen. Der Verschlußkörper 407 sitzt in seiner in Fig. 6 gezeigten zweiten, unteren Stellung auf einem Anschlag 410 auf, der etwa aus einem zu dem zylindrischen Hohlraum 406 quer verlaufenden Stahldraht 411 mit einer bewegungsdämpfenden Beschichtung 412 aus etwa Silikonmaterial bestehen kann. Der Verschlußkörper 407 besteht vorzugsweise aus einem permanentmagnetischen Material.

Koaxial zu der Achse des zylindrischen Hohlraums 406 ist eine Magnetspule 413 angeordnet, die über eine elektrische Zuleitung 414 gespeist werden kann.

In der Seitenwand des zylindrischen Hohlraumes 406 sind unterhalb der Höhe D Aussparungen 415 und 416 (Fig. 6) vorgesehen, die in der zweiten, unteren in Fig. 6 gezeigten Stellung des Verschlußkörpers 407 in Verbindung mit dem Innenraum 406 des zylindrischen Hohlraums und am unteren Ende mit einer Auslaßleitung 417 stehen. Die Auslaßleitung ist an ihrem unteren Ende in Form einer Einstichkanüle 418 ausgebildet, die etwa den Verschlußstopfen 419 eines nicht näher dargestellten Analyseprobenröhrchens durchstechen kann. Koaxial zu dem Einstichrohr 418 ist ein ringförmiger Vorsprung 420 ausgebildet, der gegen die Oberseite 421 des Verschlußstopfens 419 dichtend zur Anlage kommt. Auf diese Weise wird zwischen diesem ringförmigen Vorsprung 420 und dem Verschlußstopfen ein Ringraum 422 gebildet, der über eine Ausnehmung 423 mit einer Leitung 434 in Verbindung steht. Diese Leitung 434 kann die in Fig. 2 dargestellte Leitung 34 oder die in Fig. 3 dargestellte Leitung 134 sein, die mit dem Melkvakuum in Verbindung steht. Auf diese Weise liegt in dem Ringraum 422 nach dem Einstechen des Endes 418 in den Verschlußstopfen 419 Melkvakuum an, das einerseits verhindert, daß sich über die Abdichtung des Stopfens 419 ein Druckanstieg in der Analysenprobenflasche ergibt und daß andererseits auch das Gasvolumen im Inneren der Analysenprobenflasche, das durch die eintretende Milch verdrängt wird, abgesaugt werden kann, so daß sichergestellt ist, daß eine Probenentnahme unter Gleichdruck erfolgt.

Das Ventil könnte ggf. auch etwa als Quetschventil ausgebildet sein, bei dem etwa ein Schlauch jeweils abgequetscht bzw. freigegeben wird.

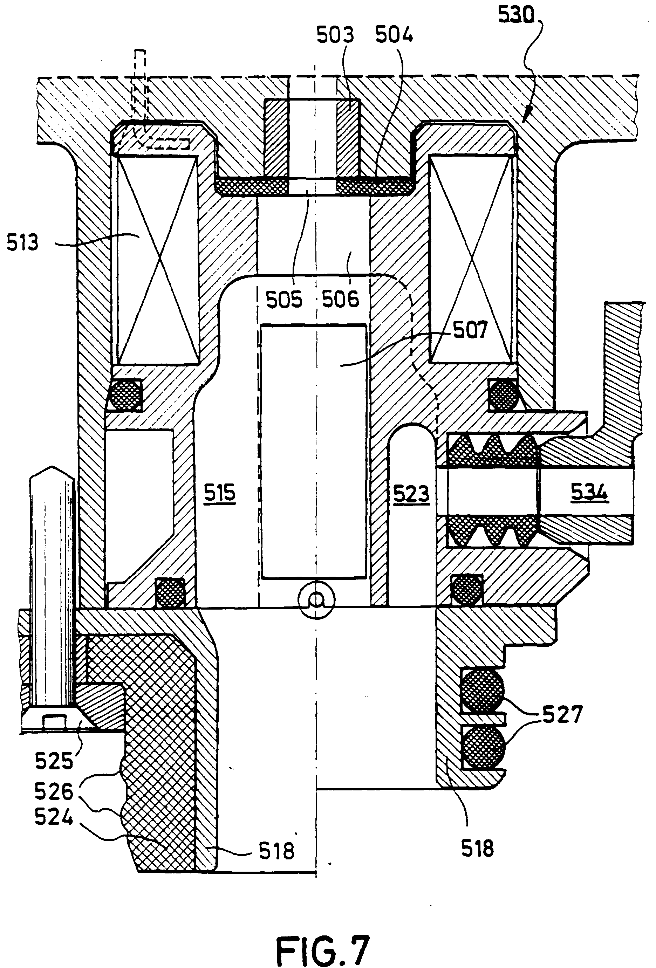

In Fig. 7 ist eine ähnliche Ausführungsform einer Milchprobenentnahmeeinrichtung gezeigt, weshalb gleiche Teile mit den gleichen, jedoch um 100 erhöhten Bezugszeichen bezeichnet sind. In dieser Figur ist lediglich in der linken unteren Hälfte eine weitere Ausführungsform gezeigt, in der ein Pfropfen 524 auf das untere Rohrende 518 aufgesetzt und mit einer Schraube 525 am Gehäuse 530 befestigt werden kann. Auf diesem Pfropfen 524 kann sodann ein entsprechendes rohrförmiges Analyseprobengefäß aufgesetzt werden, das mit seinem inneren Umfang sodann dichtend gegen die Wülste 526 anliegt.

Auf der unteren rechten Hälfte der Fig. 7 ist eine andere Ausführungsform dargestellt, bei der auf der Außenseite des Rohres 518 zwei O-förmige Dichtungsringe 527 gehaltert sind, auf die sodann dichtend ein Analyseprobenbehälter direkt aufgeschoben werden kann.

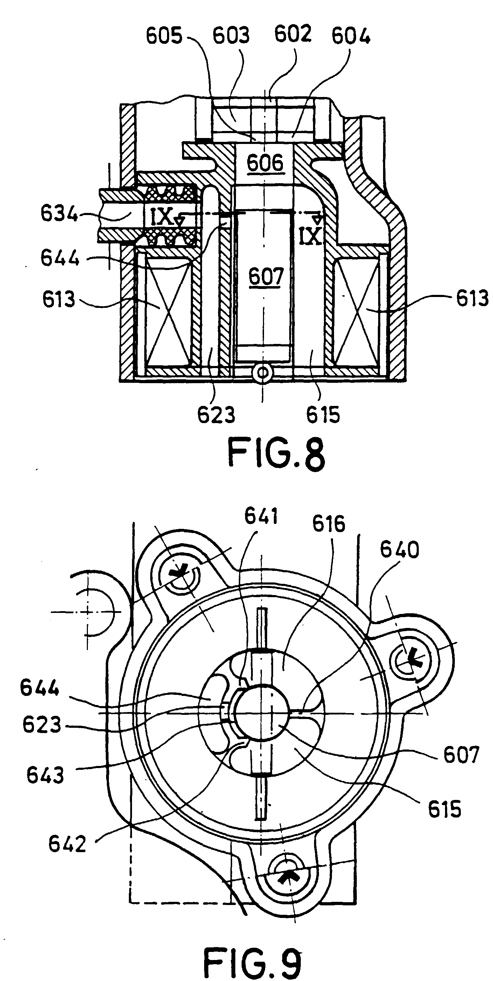

In den Figuren 8 und 9 ist eine weitere, leicht abgewandelte Ausführungsform einer Milchprobenentnahmeeinrichtung entsprechend Fig. 6 dargestellt, weshalb gleiche Bauteile mit den gleichen Bezugszeichen wie in Fig. 6, jedoch um 200 erhöht bezeichnet sind. Bei dieser Ausführungsform ist die Magnetspule 613 zu dem unteren Ende des sich in der zweiten unteren Stellung befindlichen Verschlußkörpers 607 hin verlegt, was sich als äußerst stabilitätsfördernd für ein exaktes Verschließ- und Öffnungsverhaltens des Verschlußkörpers erwiesen hat. Der Verschlußkörper ist hier auf lediglich drei Führungsstegen 640, 641 und 642 geführt. Zwischen dem Steg 640 und den Stegen 641 bzw. 642 sind jeweils die bereits beschriebenen Ausnehmungen 615 und 616 für die Ableitung der Milch vorgesehen. Zusätzlich ist zwischen den Stegen 641 und 642 eine demgegenüber flachere Ausnehmung 643 ausgebildet, die über eine weitere Ausnehmung 644 mit der Kammer 623 in Verbindung steht, die unter Melkvakuum gehalten wird. Die Ausnehmung 644 bewirkt, daß auch das obere Ende des Verschlußkörpers 607 in der zweiten unteren Öffnungsstellung unter Vakuum steht, so daß ein reibungsloses Abfließen der Milch erzielt wird (keine Pipettenwirkung).

Die Abspaltung von Milch mit den in den Figuren 2 bis 4 und 6 bis 9 beschriebenen Milchprobenentnahmeeinrichtungen erfolgt bevorzugt derart, daß die Anordnung als ein Auf-Zu-Ventil arbeitet. Wird etwa, ausgehend von der in Fig. 6 gezeigten zweiten unteren Stellung des Verschlußkörpers 407 ein elektrischer Impuls mit geeigneter Stromrichtung und Stärke auf die Magnetspule 413 gegeben, so wird der Verschlußkörper 407 nach aufwärts bewegt bis er gegen die Prallplatte 404 zur Anlage kommt. In dieser ersten oberen Stellung, die gleichzeitig der Verschlußstellung entspricht, wird der Zufluß von Milch über die Leitung 402 durch den Verschlußkörper 407 unterbrochen. Da entweder der Verschlußkörper 407 oder der Ring 403 aus einem Permanentmagneten bestehen, während jeweils das andere Teil aus einem ferromagnetischen Material besteht, wird der Verschlußkörper 407 in dieser Stellung gehalten, auch wenn die Magnetspule 413 keinen Strom mehr führt. Zur Öffnung des Ventils braucht lediglich ein etwa gleich großer Impuls mit entgegengesetzter Stromrichtung durch die Magnetspule 413 geschickt zu werden, um die magnetischen Haltekräfte zwischen dem Ring 403 und dem Verschlußkörper 407 zu überwinden und den Verschlußkörper wieder in seine zweite untere Offenstellung zurückzubringen. In dieser Stellung wird der Milchfluß durch die Leitung 402 freigegeben. In dieser zweiten unteren Stellung bedarf es gleichfalls keiner Haltekraft der Magnetspule für den Verschlußkörper 407, da dieser auf der Auflage 410 ruht. Da der Verschlußkörper aus einem äußerst kleinen Körper von lediglich etwa 6 mm Durchmesser und etwa 16 mm Länge, der einen Hub von etwa 8 mm hat, bestehen kann, während die zu verschließende Leitung 402 etwa einen Durchmesser zwischen 1,5 und 3 mm hat, kann die träge Masse des Verschlußkörpers 407 äußerst niedrig gehalten werden, da das Gesamtvolumen des Verschlußkörpers unter 1,35 ml und unter einem Gewicht von 10 g liegt. Es bedarf hierdurch lediglich kürzester Impulse von 10 bis 100 ms Länge und mit einer maximalen Leistung von etwa 1,5 Watt, um das Ventil zu öffnen oder zu schließen. Das bedeutet, daß selbst bei sehr hohen Betätigungszyklen der mittlere Energieverbrauch äußerst niedrig (typisch 0,2 Watt) ist, da zwischen den Impulsen keine Energie verbraucht wird. Weit bedeutsamer jedoch ist noch, daß mit einem solchen Ventil definierte Öffnungszeiten bis hinab zu 0,05 Sekunden Öffnungszeit bei einem definierten Abspaltfluß erreichbar sind.

Zur Steuerung des Ventils werden vorzugsweise die Impulse mit entgegengesetzter Stromrichtung derart auf die Magnetspule aufgegeben, daß die Spannung an der Magnetspule umgepolt wird. Gemäß einer zweckmäßigen Ausgestaltung könnte die Anordnung aber auch so getroffen sein, daß statt einer Magnetspule zwei Magnetspulen vorgesehen sind, die gegensinnig gewickelt sind, wobei abwechselnd der Impuls auf die erste bzw. die zweite Magnetspule gegeben wird, um den Verschlußkörper von der ersten in seine zweite Stellung und zurück zu bewegen.

Obwohl die Vorrichtung vorstehend vorzugsweise in Verbindung mit einer Impulssteuerung beschrieben wurde, könnte dennoch die Vorrichtung ohne Verwendung eines Permanentmagneten und lediglich unter Verwendung eines ferromagnetischen Materials für den Verschlußkörper derart durchgeführt werden, daß auf die Magnetspule jeweils ein Strom in eine erste Richtung gegeben wird, der so lange fließt, wie der Verschlußkörper in einer seiner Stellungen gehalten werden soll. Zur Bewegung des Verschlußkörpers in seine andere Stellung wird sodann die Stromrichtung umgepolt und der Strom vorzugsweise wiederum so lange gehalten, bis eine Umschaltung erfolgt. Eine solche Verfahrensweise würde jedoch den Stromverbrauch und die Wärmebelastung der Spule wesentlich erhöhen.

Bei der in Fig. 6 gezeigten Ausführungsform ergibt sich ein gewisser Kolbeneffekt während sich der Verschlußkörper über die Strecke D bewegt. Ein solcher Kolbeneffekt ist für die Wirkung des Ventils nicht unbedingt erforderlich, jedoch hat er sich als äußerst vorteilhaft erwiesen.

Die Wirkung des Kolbeneffektes, der in das Ventil eingebaut werden kann, ist wie folgt: Beim Schließen des Verschlußkörpers fährt dessen Kopfteil in den zylinderförmigen Hohlraum 406 der Länge D einem Kolben gleich ein. Dabei verdrängt er die in diesem Zylinderraum befindliche Milch mit der Folge, daß der Abspaltkanal 402 von rückwärts her freigeblasen wird, wodurch die Milch für den nächstfolgenden Abspaltzyklus optimal ausgetauscht wird. Dies verbessert die Repräsentativität noch zusätzlich. Außerdem bewirkt dieses Freiblasen durch den Kolbeneffekt eine Reinigung des gesamten Abspaltkanals inklusive Blende 31, 131 von eventuellen Schmutzpartikeln vor jedem Abspaltzyklus. Dieser Effekt kann in der Reinigungsphase des Geräts durch eine dann stark erhöhte Ventilschaltfrequenz (z.B. 90 - 120 Z/min) noch weiter gesteigert und ausgenützt werden.

Bei der auf die Schließbewegung folgenden Öffnungsbewegung des Verschlußkörpers wird durch denselben Kolbeneffekt Milch aus dem Abspaltkanal angesaugt, bevor diese dann am Ende der Zylinderlänge D frei über die Kopffläche des Verschlußkörpers hinweg durch die Aussparungen 415, 416 zur Analysenflasche fließt. Das Milchvolumen, das beim Öffnen des Ventils angesaugt bzw. beim Schließen zurückgeschoben wird, ist gleich groß und hat damit keinen (unmittelbaren) Einfluß auf die Abspaltmenge. Dieses hin- und hergeschobene Milchvolumen hängt lediglich ab von der wirksamen Länge des Zylinderabschnitts D und ggf. noch von dem Ringspalt zwischen Kolben und Zylinder. Außerdem muß der Gesamthub des Verschlußkörpers deutlich größer sein als die Länge D, da nur so die Milch schließlich über die Aussparungen abfließen kann. Aufgrund der kleinen Abmessungen des Zylinders (z.B. Durchmesser 6 mm, Länge D 5 mm) wirken die Kapillar- und Kohäsionskräfte der Milch so stark, daß der Zylinder auch in vertikaler Position vor dem nächsten Schließvorgang stets mit Milch gefüllt ist. Sollte die Beschleunigungsbewegung des Verschlußkörpers beim Schließen und beim Öffnen unterschiedlich sein, könnte dies durch Anlegen einer unterschiedlichen Stromstärke an die Spule(n) für die beiden Bewegungsrichtungen korrigiert werden.

Der wichtigste Vorteil des beschriebenen Kolbeneffekts ist jedoch folgender: Zur Verwirklichung einer jederzeit mengenproportionalen Probe und eines möglichst kleinen Verschleppungsfehlers sowie auch einer möglichst kleinen Bauhöhe, soll der Abspaltkanal oberhalb der Durchflußöffnung 405 des Ventils möglichst kurz sein. Damit ergeben sich, insbesondere bei niedrigen Milchflüssen, sehr kleine hydrostatische Drücke (typisch 0,5 bis 2 cm Wassersäule). Auch die Staudrücke bei kinetischen Abspaltsystemen (siehe etwa Fig. 4 und 5) sind bei niedrigen Milchflüssen sehr klein. Bei solch geringen hydrostatischen bzw. hydrodynamischen Drücken kommen die Kapillar-, Kohäsions- und Wand-Kräfte der Milch zunehmend zum Tragen. Die Folge ist ein unregelmäßiges, unpräzises und träges Anlaufen des Abspaltstromes nach der Freigabe der Durchlaßöffnung 405 eines herkömmlichen Auf/Zu-Ventils (ohne Kolbeneffekt). Der beschriebene Kolbeneffekt wirkt hier gezielt als Hilfe zur Überwindung des trägen, unpräzisen Anlaufverhaltens des Abspaltstromes bei sehr niedrigen hydrostatischen bzw. hydrodynamischen Drücken. Erst auf diese Weise werden kurze und kürzeste Ventiloffenzeiten pro Zyklus für kleinste, aber reproduzierbare Abspaltmengen, wie sie gerade bei niedrigen Milchflüssen für eine repräsentative Probe unumgänglich nötig sind, die direkt in eine kleine Analysenflasche abgefüllt werden soll, ermöglicht.

Mit zunehmendem hydrostatischen bzw. Stau-Druck läuft der Abspaltfluß jederzeit spotan und unmittelbar an, so daß der Kolbeneffekt automatisch immer mehr ins Leere läuft, weil der Abspaltfluß der Kolbenbewegung leicht und ohne Widerstand folgen kann. Entsprechend wird das natürliche Auslaufverhalten bei höheren Drücken in keiner Weise gestört.

Die genauen Ausflußverhältnisse unter Einbeziehung des gegebenen Kolbeneffekts werden am besten empirisch ermittelt. Dazu wird etwa im Labor die Möglichkeit eingerichtet, für die konkrete Geräteanordnung unterschiedliche Niveaus von hydrostatischen oder hydrodynamischen Drücken (z.B. 0,5, 1,0, 2,0, 4,0, 8,0 cm Wassersäule) bzw. von entsprechenden Milchflüssen (z.B. 0,1, 0,25, 0,5, 1,0, 2,5, 5,0, 9,0, 12,0 l/min) einzustellen. Für jedes Niveau werden die Abspaltmengen erfaßt, die sich bei verschiedenen Kombinationen von Ventiloffenzeit pro Abspaltzyklus und Anzahl der Abspaltzyklen ergeben. Wichtig ist dabei, daß das Produkt aus Ventiloffenzeit und Zykluszahl pro Minute, d.h. die Offenzeit (s/min) konstant gehalten wird, was bedeutet, daß die theoretische Abspaltmenge für das jeweilige Niveau jeweils konstant ist.

Empirisch stellt sich jedoch heraus, daß die tatsächlichen Abspaltmengen innerhalb der einzelnen Niveaus nicht immer gleich groß sind. Insbesondere bei niedrigen Druck- bzw. Fluß-Niveaus ergeben unterschiedliche Kombinationen der Steuerungsfaktoren Ventiloffenzeit/Zyklus und Anzahl Zyklen/Minute unterschiedliche, also nicht wie erwartet konstante Abspaltmengen, auch wenn die Offenzeit immer streng konstant gehalten wird. So wird auf dem jeweils gleichen Niveau bei kurzen Ventiloffenzeiten (bei entpsrechend höherer Anzahl Zyklen/Minute) eine größere Abspaltmenge erreicht als bei längeren Ventiloffenzeiten (bei entsprechend kleinerer Anzahl Zyklen/Minute). Dieses Ergebnis hängt offenbar zusammen damit, daß mit zunehmender Zykluszahl/Minute (bei entsprechend abnehmender Ventiloffenzeit) ebenfalls häufiger der Kolbeneffekt wirksam wird, der dem bei niedrigen Niveaus träge anlaufenden Abspaltfluß bei jeder Ventilöffnung eine leichte Beschleunigungshilfe verleiht. Stellt man diese Verhältnisse als Kennfeld (Abspaltmenge; Ventiloffenzeit; Druck- bzw. Fluß-Niveau) dar, so zeigt sich, daß bei niedrigen Niveaus (z.B. Milchfluß 250 ml/min) im Bereich zwischen z.B. 0,1 und 0,25 s Ventiloffenzeit die Abspaltmenge praktisch konstant und sehr gut reproduzierbar ist. Bei zunehmend längeren Ventiloffenzeiten pro Abspaltzyklus (mit entsprechend weniger Abspaltzyklen) hingegen nimmt die Abspaltmenge in ihrer Größe zunehmend ab. Die Häufigkeit des Kolbeneffekts reicht unter diesen Bedingungen nicht mehr aus, um einen reproduzierbaren stabilen Abspaltstrom zu gewährleisten. Das Problem des Anlaufens des Abspaltflusses aufgrund seiner Trägheit nimmt mit zunehmend höheren Milchflüssen bzw. Drücken zunehmend ab, so daß schließlich innerhalb der höheren Niveaus alle technisch möglichen Kombinationen von Ventiloffenzeit und Anzahl Zyklen/Minute eine konstante und sehr gut reproduzierbare Abspaltmenge ergeben, die der theoretischen Berechnung entspricht. Damit ist bei den höhereren Niveaus aus dieser Sicht keine Bereichsbeschränkung für die Ventilsteuerungsfaktoren mehr nötig.

In bezug auf die Häufigkeit einer Schließ- und Öffnungsbewegung wurde festgestellt, daß 120 Zyklen pro Minute mit definierter Offenzeit und Geschlossenzeit, die zusammen die Zykluszeit ergeben, durchaus realisierbar sind bei definiertem Abspaltfluß. Eine derart hohe Zykluszahl hat jedoch den Nachteil, daß ein erhöhter Verschleiß einsetzt und der hierbei erzeugte Geräuschpegel verhältnismäßig hoch ist. Bevorzugt wird deshalb das Ventil bei einer niedrigeren Zykluszahl pro Zeit, etwa unter 30 Zyklen pro Minute, betrieben.

Um eine möglichst repräsentative und verschleppungsarme Probe unter allen Milchflußbedingungen ziehen zu können, sollte dafür Sorge getragen werden, daß das Volumen der Zuleitung 402 möglichst klein und in etwa der Größenordnung der pro Öffnung abzuspaltenden Milchmenge gehalten wird, oder daß in dem Volumen 402 ein ständiger Milchaustausch entsprechend dem Gesamtmilchfluß stattfindet. In diesem Zusammenhang bringt die Milchprobenentnahmevorrichtung den wesentlichen Vorteil mit sich, daß aufgrund der möglichen kurzen Öffnungszeiten der Querschnitt der Zuführleitung 402 verhältnismäßig groß gewählt werden kann, so daß an sich verhältnismäßig große Abspaltflüsse ermöglicht werden und daß dennoch eine repräsentative Probe erzielt werden kann, da bei jedem Schließ- und Öffnungsvorgang die Milch aus der Leitung 402 zurückgepumpt und neue angesaugt wird, so daß jeweils frische Milch aus dem jeweiligen Milchfluß abgespalten wird.

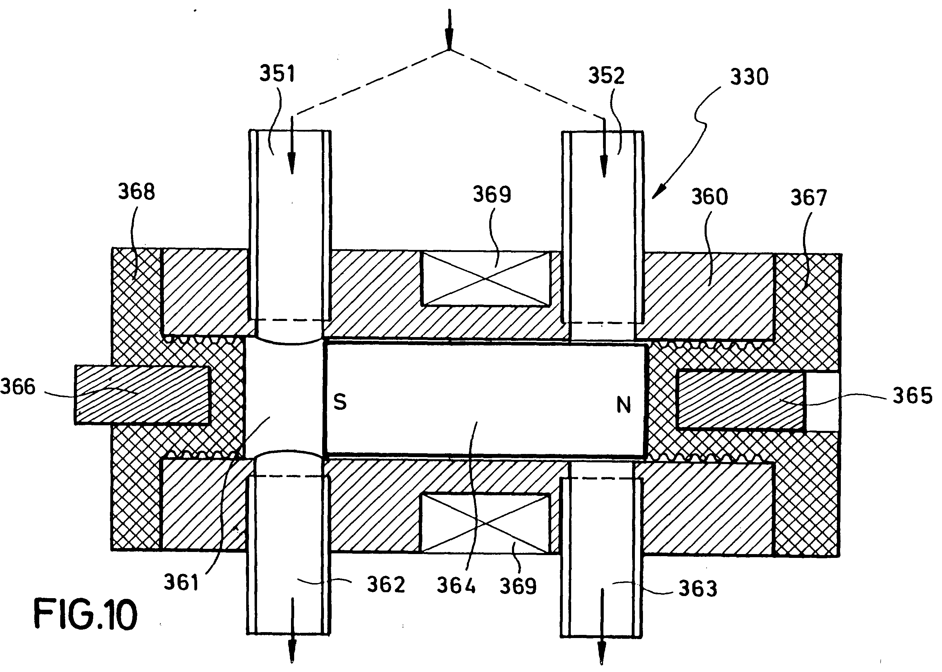

In der Fig. 10 ist die in Fig. 5 schematisch gezeigte Milchprobenentnahmeeinrichtung 330 im einzelnen näher dargestellt. Die Leitungen 351 und 352 münden in einem in dem Gehäuse 360 ausgebildeten, im wesentlichen zylindrischen Hohlraum 361. Auf den in Mündungen der Leitungen 351 und 352 gegenüberliegenden Seitenwänden des Hohlraums sind mit den erstgenannten Leitungen fluchtende Abflußleitungen 362, 363 vorgesehen. In dem Hohlraum 361 ist ein ebenfalls zylindrischer Permanentmagnet 364 vorgesehen, der im wesentlichen die gleiche Querschnittsfläche wie der Hohlraum 361 aufweist. Der Permanentmagnet ist in dem Hohlraum 361 entlang dessen Achse zwischen einer ersten in Fig. 11 gezeigten Stellung, in der sein rechtes Ende die Enden der Leitungen 352 und 363 verschließt und die Enden der Leitungen 351 und 362 freigibt, und einer zweiten, in Fig. 11 nicht gezeigten, Stellung verschiebbar, in der sein linkes Ende in Fig. 11 die Enden der Leitung 351 und 362 verschließt, während er die Enden der Leitungen 352 und 363 freigibt. In dem Gehäuse 360 sind auf der Achse des zylindrischen Permanentmagneten 364 jeweils im Abstand zu seiner ersten bzw. zu seiner zweiten Stellung zwei ferromagnetische Körper 365 und 366 angeordnet. Die genannten Körper sind jeweils in einem stopfenförmigen Teil 367 bzw. 368 gehalten, die jeweils aus einem die Bewegung des Kerns dämpfenden Material bestehen. Bei einer Bewegung des Permanentmagneten 364 von seiner ersten in seine zweite Stellung und zurück kommt dieser jeweils gegen diese stopfenförmige Teile zur Anlage. Koaxial zu der Achse des Permanentmagneten 364 ist eine elektromagnetische Spule 369 angeordnet. Mit Hilfe von vorzugsweise impulsförmigen Strömen von entsprechender Größe, die durch die Elektromagnetspule 369 geschickt werden, kann der Elektromagnet von seiner ersten in seine zweite und von seiner zweiten zurück in seine erste Stellung bewegt werden, indem der Permanentmagnet sodann jeweils durch magnetische Kräfte an dem ferromagnetischen Körper 366 bzw. 365 gehalten wird, ohne daß durch die elektromagnetische Spule weiter Strom fließt.

Die in Fig. 10 in Verbindung mit Fig. 5 gezeigte Milchprobenentnahmevorrichtung weist den Vorteil auf, daß aus dem jeweiligen Milchstrom kontinuierlich ein Anteil abgespalten wird, der durch die Abzweigung 350, die Leitungen 351 und 362 zurück in den Milchsumpf 325 fließt, wenn sich der Permanentmagnet 364 in der in Fig. 11 gezeigten Stellung befindet. Dieser Strom wird unterbrochen, wenn der Permanentmagnet in seine zweite Stellung bewegt wird, während sodann ein Milchprobenabspaltstrom über die Leitungen 352 und 363 in den Analysenprobenbehälter fließen kann. Indem das Volumen der Abzweigung 350 und den Leitungen 351 und 352 möglichst klein gehalten wird, ist gewährleistet, daß sich in der zu dem Hohlraum 361 führenden Leitung 351 praktisch immer Milch entsprechend dem jeweiligen Milchfluß befindet, wodurch eine repräsentative Probenentnahme gewährleistet ist und Anlaufprobleme des Abspaltstromes bei niedrigen Staudrücken nicht auftreten.

Zur Durchführung des erfindungsgemäßen Verfahren unter Verwendung einer Milchprobenentnahmeeinrichtung bzw. eines Ventils der vorstehend beschriebenen Art wird ausgegangen von den folgenden Überlegungen und Festlegungen:

a) In den Analyseprobenbehälter soll jeweils eine Menge zwischen 20 bis 40 ml Milch abgespalten werden, unabhängig von dem Erwartungswert E, d.h. der Gesamtmilchmenge in kg (bzw. in ml) der zu melkenden Kuh. Für die Überlegungen wird deshalb von einem abzuspaltenden Probengesamtvolumen von 30 ml ausgegangen,

b) weiterhin sollen die abgespaltenen Teilmengen proportional zum jeweiligen Milchfluß genommen werden.

Unter diesen Annahmen folgt, daß in Abhängigkeit von dem Milchfluß ein

$$\text{Abspaltvolumen/Zeit (ml/min)} =$$

$$\frac{\text{Gesamtprobe (ml)}}{\text{Erwartungswert (ml)}} \text{ x Milchfluß (ml/min)} \tag{1}$$

abgespalten werden muß, um auf die Gesamtprobenmenge zu kommen.

Bestimmt man nunmehr den Abspaltfluß (ml/min) bei dauernd geöffnetem Ventil = Abspaltfluß 100 % etwa gemäß den Anordnungen der Figuren 2 und 3, so ist festzustellen, daß der Abspaltfluß 100 % (dauernd geöffnetes Ventil) zu der durch die Bodenöffnung 31 bzw. 131 mit einem bestimmten Querschnitt (A) ausfließt, eine Funktion der Stauhöhe h (bzw. des hydrostatischen Drucks) nach folgender Formel ist:

$$\text{Abspaltfluß (100 \%) [ml/min]} =$$

$$60 \text{ x } \mu \text{ x A x SQR (2 x g x h)} \tag{2}$$

wobei:

g   = Erdbeschleunigung ($cm/s^2$)
h   = Stauhöhe (cm)
A   = Querschnitt der Bodenöffnung (Blende) ($cm^2$)
$\mu$   = Ausflußbeiwert 0,63
SQR = Wurzel aus

bedeuten.

Wenn sich die Stauhöhe h, d.h. der hydrostatische Druck mit sich änderndem Milchfluß nicht ändert, ist der Wert des Abspaltflusses (100 %) eine Konstante. So ergibt sich z.B. für eine konstante Stauhöhe von h = 2 cm und einem Blendendurchmesser von 0,15 cm, woraus der Querschnitt der Bodenöffnung zu A = 0,0176 $cm^2$ folgt, ein konstanter Abspaltfluß (100 %) = 41,67 ml/min.

Wird die Größe des Milchflusses in einem Staugefäß über die Stauhöhe (bzw. den hydrostatischen Druck) vor einem vertikal verlaufenden Meßschlitz von konstanter Breite S gemessen (etwa wie bei dem in Fig. 2 gezeigten Ausführungsbeispiel), so besteht folgender Zusammenhang:

$$\text{Milchfluß (ml/min)} =$$

$$60 \text{ x } \mu \text{ x S x 2/3 x SQR[2 x g] x } h^{3/2} \tag{3}$$

wobei:

g   = Erdbeschleunigung ($cm/s^2$)
h   = Stauhöhe (cm)
S   = Schlitzbreite (konstant) (cm)
$\mu$   = Ausflußbeiwert 0,63
SQR = Wurzel aus

bedeuten.

In Verbindung mit der Gleichung (2) läßt sich daraus der vom Milchfluß abhängige Abspaltfluß (100 %) bestimmen zu:

Abspaltfluß (100 %) =

$$60 \times \mu \times A \times SQR(2 \times g) \times [\text{Milchfluß})/<60 \times \mu \times S \times 2/3 \times SQR(2 \times g)>]^{1/3} \qquad (4)$$

Bei einer beispielsweise angenommenen konstanstanten Schlitzbreite von S = 0,25 cm und einem Blendenquerschnitt von A = 0,0176 cm² (= 1,5 mm Blendendurchmesser) errechnen sich daraus folgende theoretischen Tabellenwerte:

Tabelle 1

| gemessener Milchfluß (ml/min) | Abspaltfluß (100 %) (Ventil dauernd offen) (ml/min) |
| --- | --- |
| 100 | 20,93 |
| 250 | 28,41 |
| 500 | 35,78 |
| 1000 | 45,07 |
| 2500 | 61,15 |
| 5000 | 77,03 |
| 9000 | 93,68 |
| 12000 | 101,95 |

Selbstverständlich läßt sich eine Tabelle dieser Art auch als empirisch gemessene Kennlinie dastellen. Aus dieser Tabelle läßt sich nunmehr die benötigte Offenzeit des Ventils in Sekunden/Minute bestimmten, um das eingangs angegebene Abspaltvolumen/Zeit in ml/min zu erhalten:

$$\text{Offenzeit (s/min)} = \frac{(\text{Abspaltvolumen (ml/min) x 60 s})}{(\text{Abspaltfluß 100 %) (ml/min)}} \qquad (5)$$

Daraus ergäbe sich nach Tabelle 2 eine benötigte Offenzeit in Sekunden/ Minute (bei konstanter Stauhöhe).

Tabelle 2

| (benötigte Offenzeit in sec/min bei konstanter Stauhöhe) | | | | |
| --- | --- | --- | --- | --- |
| | Erwartungsmenge | 30.000 ml | 10.000 ml | 6.000 ml |
| Milchfluß (ml/min) | | | | |
| 100 | | 0,14 | 0,43 | 0,72 |
| 2500 | | 3,60 | 10,79 | 17,99 |
| 12000 | | 17,28 | 51,84 | 86,39 |

Hieraus ergibt sich ein Verhältnis von maximaler : minimaler Offenzeit von 86,39 : 0,14 = 617 : 1.

Da Werte größer als 60 s/min nicht möglich sind, würde dies bedeuten, daß der konstante Abspaltfluß (hier 41,67 ml/min) um mindestens den Faktor 1,5 vergrößert werden müßte, um zu realen Offenzeiten zu kommen. Damit würde aber die kürzeste Offenzeit auf unter 0,1 s/min fallen, was einzelne Ventiloffenzeiten pro Zyklus von weit weniger als 0,1 sec bedingen würde, und damit technisch nur noch sehr schwer realisierbar wäre.

Bei variabler Stauhöhe ergäben sich demgegenüber die folgenden benötigten Offenzeiten in s/min:

Tabelle 3

| (benötigte Offenzeit in sec/min bei variabler Stauhöhe) | | | | |
| --- | --- | --- | --- | --- |
| | Erwartungsmenge | 30.000 ml | 10.000 ml | 6.000 ml |
| Milchfluß (ml/min) | | | | |
| 100 | | 0,29 | 0,86 | 1,43 |
| 250 | | 0,53 | 1,58 | 2,64 |
| 500 | | 0,84 | 2,52 | 4,19 |
| 1000 | | 1,33 | 3,99 | 6,66 |

Tabelle 3   (fortgesetzt)

| (benötigte Offenzeit in sec/min bei variabler Stauhöhe) | | | | |
|---|---|---|---|---|
| | Erwartungsmenge | 30.000 ml | 10.000 ml | 6.000 ml |
| Milchfluß (ml/min) | | | | |
| 2500 | | 2,45 | 7,36 | 12,26 |
| 5000 | | 3,89 | 11,68 | 19,47 |
| 9000 | | 5,76 | 17,29 | 28,82 |
| 12000 | | 7,06 | 21,19 | 35,31 |

Daraus ergäbe sich ein Verhältnis von maximaler : minimaler Offenzeit von 35,31 : 0,29 = 122 :1.

Wollte man nach diesen theoretischen Offenzeiten vorgehen, so bestände die Schwierigkeit darin, daß sich etwa bei hohen Milchflüssen und niedrigen Erwartungsmengen verhältnismäßig lange Offenzeiten ergeben, während der sich der Milchfluß selbstverständlich bereits gravierend geändert haben kann, so daß keine repräsentative Probe entnommen würde. Andererseits ergibt sich bei niedrigen Milchflüssen und hohen Erwartungsmengen eine sehr kurze Offenzeit, während der nicht mehr ohne weiteres feststeht, daß auch die angenommene Proportionalität zwischen Abspaltvolumen/Zeit überhaupt noch besteht.

Gemäß der Erfindung wird deshalb nicht mit einer Offenzeit in s/min gesteuert, sondern diese Offenzeit wird in mehrere Probenahmezyklen mit entsprechend kürzeren Ventiloffenzeiten pro Zyklus aufgeteilt, dabei wird so vorgegangen, daß die jeweilige Offenzeit des Ventils lediglich in einem begrenzten Bereich variiert wird, in dem feststeht, daß der Abspaltfluß proportional zur Zeit ist. Die eigentliche Steuerung erfolgt in erster Linie zunächst dadurch, daß das pro Minute zu entnehmende Probevolumen in mehreren Zyklen mit jeweils einer kürzeren Ventiloffenzeit pro Abspaltzyklus entnommen wird, wobei ein Zyklus jeweils aus der Ventiloffenzeit und der Ventil-Nichtoffenzeit pro Abspaltzyklus besteht. Danach läßt sich die Anzahl der Abspaltzyklen, mit der die Steuerung durchgeführt wird, bestimmen aus:

$$\text{Offenzeit (s/min)} =$$

$$\text{Anzahl der Abspaltzyklen (n/min) x Ventiloffenzeit}$$

$$\text{pro Abspaltzyklus (s)} \tag{6}$$

Nun ist die Anzahl der möglichen Abspaltzyklen (n/min), die sich mit einem Ventil der beschriebenen Art realisieren lassen, begrenzt. Zwar lassen sich nach oben hin ohne weiteres Abspaltzyklen von n = 120 leicht erreichen. Aus Gründen des Verschleisses und des hohen Geräuschpegels wird die Zahl der Abspaltzyklen jedoch vorzugsweise auf n = 30/min begrenzt. Nach unten hin ist die Zahl der Abspaltzyklen ebenfalls dadurch begrenzt, daß auch zum Melkende hin bei niedrigen Milchflüssen noch repräsentative Proben entnommen werden müssen. Dies ist besonders wichtig, da sich die Milchinhaltsstoffe, insbesondere der Fettgehalt der Milch, gegen Ende der Melkphase hin beträchtlich ändern. So ist der Fettgehalt gegen Melkende hin wesentlich größer als zu Anfang der Melkphase. Daraus folgt, daß die Zahl der Abspaltzyklen nicht unter 2 bis 3 pro Minute sinken sollte.

Wird nun etwa entsprechend der in Fig. 3 gezeigten Ausführungsform mit einem konstanten hydrostatischen Druck gemessen, kann es wegen des großen abzudeckenden Variationsbereichs nicht möglich sein, eine ausreichende Variation allein aufgrund der Veränderungen der Zykluszahl pro Minute bei einer konstanten Ventilabspaltzeit durchzuführen. Vielmehr muß sodann bei Erreichen eines Grenzwertes für die Zykluszahl pro Zeit eine Umschaltung zu niederen oder höheren Ventiloffenzeiten erfolgen. Bei der Messung mit einem konstanten hydrostatischen Druck sollte die den hydrostatischen Druck erzeugende Milchhöhe nicht zu groß sein, um bei allen Milchflüssen (insbesondere gegen Melkende hin) eine repräsentative Probennahme zu ermöglichen. Bei niedrigen hydrostatischen Drücken ergibt sich aber, daß der Abspaltfluß nur über einen geringeren Ventiloffenzeitbereich konstant ist, da hier insbesondere der Einfluß von Kapillar- und Kohäsionskräften von Bedeutung wird. Bei dem Arbeiten unter einem konstanten, niedrigen hydrostatischen Druck gemäß Fig. 3 kann es deshalb notwendig sein, um einen großen Bereich von Ventiloffenzeiten zu ermöglichen, eine Eichkurve von Abspaltvolumen pro Ventiloffenzeit zu erstellen, die sodann in den Prozessor zur Berechnung der notwendigen Ventiloffenzeit pro Zyklus eingegeben wird.

Die Aufgabe, den gesamten Meßbereich mit einem einzigen Probenentnahmegerät abzudecken, läßt sich wesentlich vorteilhafter dann ausführen, wenn die Milchprobenentnahmevorrichtung in einer Anordnung gemäß der Fig. 2 mit sich in Abhängigkeit von dem Milchfluß ändernder Stauhöhe betrieben wird. In diesem Fall ergibt sich bereits, wie aus Tabelle 3 zu entnehmen ist, eine Verringerung des Verhältnisses Offenzeit maximal : minimal auf ein Verhältnis von lediglich 122 : 1. Das heißt, es erfolgt bereits eine Untersetzung des Meßbereiches, indem der jeweilige Milchfluß bereits als Stauhöhe, d.h. als hydrostatischer Druck, und damit zur Untersetzung der jeweiligen Offenzeiten in Abhängigkeit vom Milchfluß miteinbezogen wird. In diesem Fall kann der Bereich von Ventiloffenzeiten pro Zyklus jeweils auf einen geringeren Bereich, etwa von 0,1 bis 0,8 Sekunden begrenzt werden, in dem sichergestellt ist, daß der Abspaltfluß proportional zur Zeit ist. Wie bereits oben ausgeführt wurde, ergibt sich aber auch in diesem Fall die Notwendigkeit, daß bei kleineren Milchflüssen, was einer niedrigeren Stauhöhe bzw. hydrostatischem Druck entspricht, ein schmalerer Ventiloffenzeitraum gewählt wird, um eine Proportionalität zwischen Abspaltfluß und Offenzeit zu gewährleisten. Wie aus Tabelle 4 hervorgeht,

Tabelle 4

| Milchfluß (ml/min) | zulässige Anzahl Abspaltzyklen (n/min) (Min. bis Max.) | zulässige Offenzeit pro Abspaltzyklus (s) (Min. bis Max.) |
|---|---|---|
| < 250 | 2 - 4 | 0,10 - 0,25 |
| 800 | v | 0,10 - 0,60 |
| 1200 | v | 0,10 - 0,80 |
| v | v | v |
| > 9000 | 10 - 30 | 0,10 - > 2 |

sind in Abhängigkeit von dem jeweils gemessenen Milchfluß unterschiedliche Bereiche der Anzahl der Abspaltzyklen sowie der zulässigen Ventiloffenzeit pro Zyklus vorgesehen. So ergibt sich für Milchflüsse unter 250 ml/min eine minimale Abspaltzyklenzahl von 2 bis 4 pro Minute, während die zulässige Ventiloffenzeit pro Abspaltzyklus zwischen 0,1 und 0,25 Sekunden liegt. Der Grund für diesen eingeengten Ventiloffenzeit/Zyklus-Bereich liegt darin, daß mit längeren Ventiloffenzeiten die Anzahl der Abspaltzyklen und damit auch der Kolbeneffekt des Ventils entsprechend abnimmt. Entsprechend größere Bereiche ergeben sich für höhere Milchflüsse. Die Werte der Ventiloffenzeit pro Abspaltzyklus können programmtechnisch in 0,02 Sekundenschritten aufgelöst werden. Ergibt sich eine Umschaltung von etwa einer an die Grenze ihres Bereiches gelangten Anzahl von Abspaltzyklen durch Änderung der bis dahin verwandten Ventiloffenzeit pro Abspaltzyklus, so erfolgt die Umwandlung derart, daß die Probenzykluszeit im selben Verhältnis herauf- bzw. herabgesetzt wird wie die Ventiloffenzeit pro Abspaltzyklus.

Die jeweiligen Steuerbereiche, etwa gemäß der Tabelle 4, werden als Bereichsdaten dem Prozessor vorab eingegeben. Die Berechnung der jeweiligen Anzahl von Abspaltzyklen bei festgehaltener Ventiloffenzeit pro Abspaltzyklus wird jeweils in Abhängigkeit von dem gemessenen Milchfluß durch den Prozessor berechnet. Bei Erreichen einer entsprechenden Bereichsgrenze erfolgt eine entsprechende Umschaltung der Anzahl der Abspaltzyklen in Verbindung mit einer entsprechenden Änderung der Ventiloffenzeit pro Abspaltzyklus. Selbstverständlich kann eine solche Umschaltung auch bereits vor Erreichen der entsprechenden Bereichsgrenze erfolgen, um möglichst in einem Optimalverhältnis von Zykluszahl und Ventiloffenzeit zu bleiben.

Die vorstehenden Angaben sind nur beispielhaft und beziehen sich jeweils auf einen Durchlaßquerschnitt der Durchlaßöffnung 31 bzw. 131 von 0,0176 cm$^2$.

Im folgenden soll nochmals anhand eines Beispiels aufgezeigt werden, wie die Anzahl von Abspaltzyklen und die Ventiloffenzeit pro Abspaltzyklus berechnet und gewählt werden:

Der vorgegebene Erwartungswert E (Gesamtmilchmenge) sei 10.000 ml.

Das gewünschte Gesamtprobenvolumen sei 30 ml.

Der gemessene momentane Milchfluß sei 2.500 ml/min.

Aus Gleichung (1) läßt sich damit ein notwendiges Abspaltvolumen pro Minute von 7,5 ml/min errechnen. Aus Gleichung (4) läßt sich sodann ein Abspaltfluß (100 %) zu 61,15 ml/min berechnen.

Aus Gleichung (5) ergibt sich sodann die Offenzeit zu 7,36 s/min. Aus Tabelle 4 läßt sich bei dem angenommenen momentanen Milchfluß die Anzahl der Abspaltzyklen pro Minute zu 15 wählen. Damit ergibt sich die Ventiloffenzeit pro Zyklus aus Gleichung (6) zu 0,49 Sekunden pro Zyklus. Diese Ventiloffenzeit pro Abspaltzyklus ist nach Tabelle 4 zulässig.

Das Programm zur Steuerung der Probenentnahme kann noch dadurch weiter verfeinert werden, wenn berücksichtigt wird, daß etwa mit dem in den Abbildungen 2 bis 5 gezeigten Milchflußmesser auch jeweils die bereits geflossene Milchmenge aufaddiert und somit jeweils die in Zeitabständen geflossene Milchmenge genauer bestimmt werden kann. Wenn deshalb bei einer Melkphase der Milchfluß mit einem zunächst niedrigen Wert von etwa 200 ml/min beginnt, würde sich daraus gegebenenfalls eine Zykluszeit von 30 Sekunden einstellen. Steigt sodann verhältnismäßig schlagartig der Milchfluß, so könnte die gezogene Analysenprobe verfälscht werden. Da bei der genannten Einstellung eine Probenabspaltung erst nach 30 Sekunden stattfinden würde, wobei davon ausgegangen würde, daß in dieser Zeit lediglich ein Milchvolumen von 100 ml geflossen sei, kann man durch gleichzeitige, stetige Messung der tatsächlich geflossenen Milchmenge während eines Abspaltzyklus eine Korrektur dadurch bewirken, daß eine neue Einstellung der Zykluszeit oder der Ventiloffenzeit verlangt werden, wenn durch die Milchmengenmessung festgestellt wird, daß etwa bereits vor Ablauf der Zykluszeit von 30 Sekunden mehr als 100 ml Milch geflossen sind.

Es kann aber auch eine Steuerung derart vorgesehen werden, daß eine neue Zykluszeit oder Ventiloffenzeit ermittelt wird, wenn die Änderung des Milchflusses pro Zeit einen vorbestimmten Schwellwert übersteigt.

Eine Bestimmung der Zykluszeit und der Ventiloffenzeit läßt sich für ein mit den Anordnungen nach den Figuren 4 und 5 durchzuführendes Verfahren in gleicher Weise durchführen. Hierbei ergibt sich lediglich der Unterschied, daß der Abspaltfluß (100 %) bei ganz geöffnetem Ventil jeweils dem Fluß durch die Leitungen 241, 341 entspricht. Dieser Abspaltfluß (100 %) ist jedoch vom Verhältnis der Querschnitte der Eintrittsöffnungen dieser Leitungen zu der Milchabtransportleitung 226, 326 abhängig. Weiterhin ist dieser Abspaltfluß (100 %) vom Milchfluß abhängig und in guter Näherung reproduzierbar, aber im allgemeinen nicht mengenproportional. Der Abspaltfluß wird am besten in Form einer empirisch ermittelten Kennlinie dargestellt. Die Reinigung des Ventils kann mit einer auf ein Maximum erhöhten Zyklenzahl durchgeführt werden.

**Patentansprüche**

1. Verfahren zum Entnehmen einer für die abgemolkene Milchgesamtmenge einer Kuh repräsentativen Analysenprobe, wobei während des Abmelkvorgangs der Milch in Abhängigkeit von dem Milchfluß, d.h. der pro Zeit abgemolkenen Milchmenge, mengenproportionale Probenteilmengen entnommen werden, **dadurch gekennzeichnet**, daß unter Verwendung eines in Zyklen gesteuerten Ventils, wobei jeder Zyklus eine Ventiloffenzeit und eine Ventilgeschlossenzeit umfaßt, zur Entnahme lediglich einer unter einer vorbestimmten Maximalmenge von 50 ml gehaltenen Analyseprobe aus dem aus der Erfahrung gewonnenen Erwartungswert der abzumelkenden Milchgesamtmenge der betreffenden Kuh eine Zykluszeit und eine Ventiloffenzeit bestimmt werden, wobei Zykluszeit und Ventiloffenzeit jeweils derart gewählt werden, daß sie innerhalb vorbestimmter Wertebereiche liegen, daß die Ventiloffenzeit oder die Zykluszeit in Abhängigkeit von dem jeweils gemessenen Milchfluß gesteuert werden, und daß zur Vermeidung von außerhalb der Wertebereiche liegenden Werten der Ventiloffenzeit oder der Zykluszeit infolge der Veränderung des Milchflusses die Ventiloffenzeit und die Zykluszeit in gleichen Verhältnissen zu innerhalb der Wertebereiche liegenden Werten verändert werden.

2. Verfahren nach Anspruch 1, **dadurch gekennzeichnet**, daß die abgespaltenen Probenteilmengen unter dem gleichen Druck wie die gerade abgemolkene Milchmenge gehalten werden.

3. Verfahren nach Anspruch 1 oder 2, **dadurch gekennzeichnet**, daß dem Ventil ein aus der in der Transportleitung transportierte Milch abgetrennter, unter dem kinetischen Druck der transportierten Milch stehender Teil der Milch zugeführt wird.

4. Verfahren nach Anspruch 1 oder 2, **dadurch gekennzeichnet**, daß zur Erzeugung eines im wesentlichen von dem Milchfluß unabhängigen Abspaltflusses durch das Ventil die Milchstauhöhe über dem Ventil konstant gehalten wird.

5. Verfahren nach Anspruch 1 oder 2, **dadurch gekennzeichnet**, daß zur Änderung des Abspaltflusses durch das Ventil in Abhängigkeit vom Milchfluß über dem Ventil eine jeweils von dem Milchfluß abhängige Milchstauhöhe erzeugt wird.

6. Verfahren nach Anspruch 5, **dadurch gekennzeichnet**, daß der Wertebereich für die Ventiloffenzeit jeweils in Abhängigkeit von der gemessenen Milchstauhöhe verändert wird.

7. Verfahren nach einem der Ansprüche 1 bis 6, **dadurch gekennzeichnet**, daß der Wertebereich für die Zykluszeit zwischen 0,5 und 30 Sekunden (120 bis 2 Zyklen pro Minute) gewählt wird.

8. Verfahren nach Anspruch 7, **dadurch gekennzeichnet**, daß der Wertebereich für die Zykluszeit zwischen 2 und 30 Sekunden (30 bis 2 Zyklen pro Minute) gewählt wird.

9. Verfahren nach einem der Ansprüche 1 bis 8, **dadurch gekennzeichnet**, daß der Wertebereich für die Ventiloffenzeit zwischen 0,05 bis 2 Sekunden gewählt wird.

10. Verfahren nach Anspruch 9, **dadurch gekennzeichnet**, daß die Ventiloffenzeit zwischen 0,1 und 0,8 Sekunden gewählt wird.

11. Verfahren nach einem der vorhergehenden Ansprüche, **dadurch gekennzeichnet**, daß zur Feinkalibrierung des in der Analyseprobe enthaltenen Fettgehalts mit zunehmender Verringerung des Milchflusses gegen Melkende hin die Ventiloffenzeit zunehmend etwas vergrößert oder verkleinert wird.

12. Verfahren nach einem der Ansprüche 1 bis 11, **dadurch gekennzeichnet**, daß während jeder Schließung des Ventils ein Teil der durch das Ventil geflossenen Milch zurückgepumpt wird.

13. Verfahren nach einem der Ansprüche 1 bis 12, **dadurch gekennzeichnet**, daß bei jedem Öffnen des Ventils eine das Anlaufen des Milchflusses beschleunigende Ansaugkraft auf die Milch ausgeübt wird.

14. Vorrichtung zum Entnehmen einer für die abgemolkene Milchgesamtmenge einer Kuh repräsentativen Analysenprobe, mit einem in einer Melkleitung (4) angeordneten Milchflußmesser (6) sowie einer Prozessoreinheit (9) zur Steuerung einer mit einem Analyseprobenbehälter (8) verbundenen Milchprobenentnahmevorrichtung (7), die mit der Melkleitung in Verbindung steht, **dadurch gekennzeichnet**, daß die Milchprobenenentnahmevorrichtung (7) eine elektrisch steuerbare Magnetspule (369;, 413; 513) umfaßt, mit der ein Verschlußkörper (364; 407; 507) in eine erste, eine Durchflußöffnung (351; 405; 505) für den Probenabspaltstrom versperrende und eine zweite, diese Öffnung freigebende Stellung bewegbar ist, daß der Verschlußkörper mit einer steuerbaren Zykluszeit zwischen der ersten und zweiten Stellung hin- und herbewegbar ist, und daß die Zykluszeit und die Ventiloffenzeit, in der sich der Verschlußkörper in der die Durchflußöffnung (351; 405; 505) freigebenden zweiten Stellung befindet in Abhängigkeit von der zu erwartenden Gesamtmilchmenge jeweils derart wählbar sind, daß sie innerhalb vorbestimmter Wertebereiche liegen und wobei die Ventiloffenzeit oder die Zykluszeit in Abhängigkeit von dem jeweils gemessenen Milchfluß über die Prozessoreinheit steuerbar ist, und daß zur Vermeidung von außerhalb der Wertebereiche liegenden Werte der Ventiloffenzeit oder der Zykluszeit infolge der Veränderung des Milchflusses die Ventiloffenzeit und die Zykluszeit in gleichen Verhältnissen zu innerhalb der Wertebereiche liegenden Werten verändert werden.

15. Vorrichtung nach Anspruch 14, **dadurch gekennzeichnet**, daß der Verschlußkörper (364; 407; 507) aus einem Permanentmagneten bzw. aus einem ferromagnetischen Material besteht und daß in der Nähe der Durchflußöffnung (351; 405; 505) ein den Verschlußkörper in seiner ersten Stellung haltender Körper (366; 403; 503) aus einem ferromagnetischen Material bzw. aus einem Permanentmagneten angeordnet ist.

16. Vorrichtung nach Anspruch 14 oder 15, **dadurch gekennzeichnet**, daß der Verschlußkörper (407) aus einem zylindrischen Körper besteht, der in seitlichen Führungen (408; 640, 641, 642) geführt ist.

17. Vorrichtung nach Anspruch 15 oder 16, **dadurch gekennzeichnet**, daß der ferromagnetische Körper bzw. der aus einem Permanentmagneten bestehenden Körper ein das Ende der Durchflußöffnung für den Probenabspaltstrom bildendes Rohr (403, 503) bildet.

18. Vorrichtung nach einem der Ansprüche 15 bis 17, **dadurch gekennzeichnet**, daß der Verschlußkörper (407; 507) und/oder das dem Verschlußkörper zugewandte Ende der Durchflußöffnung (405; 505) mit einer Lage aus einem Dämpfungsmaterial (404; 504) versehen ist.

19. Vorrichtung nach einem der Ansprüche 14 bis 18, **dadurch gekennzeichnet**, daß die Magnetspule (369; 613) in Höhe der zweiten Stellung des Verschlußkörpers (364; 607) vorgesehen ist.

20. Vorrichtung nach einem der Ansprüche 14 bis 19, **dadurch gekennzeichnet**, daß die Magnetspule (369; 413; 513; 613) im Impulsbetrieb betreibbar ist.

21. Vorrichtung nach einem der Ansprüche 14 bis 20, **dadurch gekennzeichnet**, daß die Magnetspule zur Bewegung des Verschlußkörpers von der ersten in die zweite Stellung und umgekehrt jeweils mit Hilfe von Impulsen unterschiedlicher Stromrichtung steuerbar ist.

22. Vorrichtung nach Anspruch 21, **dadurch gekennzeichnet**, daß die Impulse eine Länge zwischen 10 und 100 msec aufweisen.

**23.** Vorrichtung nach einem der Ansprüche 14 bis 22, **dadurch gekennzeichnet**, daß das mit dem Analyseprobenbehälter in Verbindung stehende Ende (518) der Milchprobenentnahmeeinrichtung (530) mit dem Melkvakuum in Verbindung steht.

**24.** Vorrichtung nach einem der Ansprüche 14 bis 23, **dadurch gekennzeichnet**, daß das dem Analyseprobenbehälter zugewandte Ende der Milchprobenentnahmeeinrichtung (430) als ein in die perforierte Abdeckung (419) des Analyseprobenbehälters einführbares Rohrende (418) ausgebildet ist, und daß zwischen der Außenseite des Rohrendes (418) und einem dieses umgebenden Ringteiles (420), dessen freies Ende gegen die Oberfläche der Abdeckung (419) zur Anlage kommt, ein Ringraum (422) gebildet wird, der mit dem Melkvakuum in Verbindung steht.

**25.** Vorrichtung nach Anspruch 23, **dadurch gekennzeichnet**, daß das Ende (519) der Milchprobenentnahmeeinrichtung derart ausgebildet ist, daß der Analyseprobenbehälter dichtend auf dieses Ende aufsteckbar ist.

**26.** Vorrichtung nach einem der Ansprüche 16 bis 25, **dadurch gekennzeichnet**, daß der zylinderförmige Verschlußkörper (407; 507; 607) entlang einer im wesentlichen zylinderförmigen Führungsbahn beweglich ist, in deren dem Verschlußkörper zugewandten Fläche in Längsrichtung des Verschlußkörpers verlaufende Ausnehmungen (415, 416; 515; 615, 616, 643) vorgesehen sind, die in der zweiten Stellung des Verschlußkörpers mit der Durchflußöffnung (405; 505; 605) und dem dem Analyseprobenbehälter zugewandten Ende (418; 518) der Milchprobenentnahmevorrichtung zur Durchleitung von Milch in Verbindung steht.

**27.** Vorrichtung nach Anspruch 26, **dadurch gekennzeichnet**, daß die Verbindung zwischen der Durchflußöffnung (405; 505; 605) und den Ausnehmungen (415, 416; 515; 615, 616, 643) bei einer Verstellbewegung des Verschlußkörpers (407; 507; 607) über eine vorbestimmte Strecke (D) vor Erreichen der Verschließstellung der Durchflußöffnung bzw. während der Öffnungsbewegung des Verschlußkörpers im wesentlichen unterbrochen ist.

**28.** Vorrichtung nach einem der vorhergehenden Ansprüche, **dadurch gekennzeichnet**, daß die Durchflußöffnung (31) der Milchprobenentnahmevorrichtung (30) in einer Richtung entgegen der Richtung des Probenabspaltflusses in einen Raum (22) mündet, in dem sich eine dem jeweiligen Milchfluß entsprechende Milchstauhöhe (23) einstellt.

**29.** Vorrichtung nach Anspruch 28, **dadurch gekennzeichnet**, daß in dem Raum (22) Einrichtungen (28) zur Messung der Milchstauhöhe vorgesehen sind.

**30.** Vorrichtung nach einem der Ansprüche 14 bis 26, **dadurch gekennzeichnet**, daß die Durchflußöffnung (131) der Milchprobenentnahmevorrichtung (130) in der Richtung entgegen der Richtung des Probenabspaltflusses in einen Raum (125) mündet, in dem die abgemolkene Milch jeweils auf einer vorbestimmten Stauhöhe gehalten wird.

**31.** Vorrichtung nach einem der Ansprüche 14 bis 26, **dadurch gekennzeichnet**, daß die Durchflußöffnung der Milchprobenentnahmevorrichtung (230) in der Richtung entgegen der Richtung des Probenabspaltflusses mit einem in eine Milchtransportleitung (226) hinein vorstehenden Probenentnahmerohr (241, 341) verbunden ist.

**32.** Vorrichtung nach Anspruch 31, **dadurch gekennzeichnet**, daß das Probenentnahmerohr (241; 341) mit der Längsachse seiner Einlaßöffnung so angeordnet ist, daß diese von der Innenwand etwa im Abstand von 1/3 des Durchmessers der Milchtransport leitung (226; 326) angeordnet ist.

**33.** Vorrichtung nach einem der Ansprüche 14 bis 32, **dadurch gekennzeichnet**, daß in Richtung des Abspaltflusses vor der Durchlaßöffnung eine Zuleitung für die Milch vorgesehen ist, die sich in eine erste, die Durchlaßöffnung umfassende und mit einer Ableitung (363) in den Analyseprobenbehälter (332) verbundene Leitung (352) und eine zweite Leitung (351) verzweigt, die mit dem Milchabfluß verbunden ist, und daß der Verschlußkörper (364) derart bewegbar ist, daß er in seiner ersten Stellung die Durchlaßöffnung (352) verschließt und die zweite Leitung (351) freigibt und in seiner zweiten Stellung die Durchlaßöffnung in der ersten Leitung (352) freigibt und die zweite Leitung (351) verschließt.

## Claims

**1.** Method for extracting an analysis sample representative of the total amount of milk milked from a cow, wherein during the milking process quantitatively proportional sample partial amounts are extracted from the milk in dependence of the milk flow, i.e. the amount of milk milked per time, **characterized in that** by using a valve controlled in cycles, whereby each cycle comprises a valve opening and a valve closing time, in order to extract simply an analysis sample amount held below a predetermined maximum amount of 50 ml from the expected value of the total amount of milk to be milked from the particular cow as gained from experience, a cycle time and a valve opening time are determined, whereby the cycle time and the valve opening time are each chosen in such a way that they lie within a predetermined range of values, that the valve opening time or the cycle time are controlled in dependence of the milk flow respectively measured, and that in order to avoid values of the valve opening time or cycle time lying outside the range of values as a result of a change in the milk flow, the valve opening time and the cycle time are changed in equal ratio to the values lying within the range.

2. A method according to Claim 1, **characterized in that** the separated sample partial amounts are held under the same pressure as the amount of milk just milked from a cow.

3. A method according to Claim 1 or 2, **characterized in that** a part of the milk which is held under the kinetic pressure of the transported milk and separated from the milk transported in the milk transporting duct, is fed to the valve.

4. A method according to Claim 1 or 2, **characterized in that** for producing a separated flow through the valve which is substantially independent from the milk flow, the height of the milk build-up is held constant above the valve.

5. A method according to Claim 1 or 2, **characterized in that** above the valve, a height of milk build-up is produced for changing the separated flow through the valve in dependence of the milk flow where the height of the milk build-up is respectively dependent on the milk flow.

6. A method according to Claim 5, **characterized in that** the range of values for the valve opening time is changed respectively in dependence of the measured height of the milk build-up.

7. A method according to one of Claims 1 to 6, **characterized in that** the range of values for the cycle time is chosen between 0.5 and 30 seconds (120 to 2 cycles per minute).

8. A method according to Claim 7, **characterized in that** the range of values for the cycle time is chosen between 2 and 30 seconds (30 to 2 cycles per minute).

9. A method according to one of Claims 1 to 8, **characterized in that** the range of values for the valve opening time is chosen between 0.05 to 2 seconds.

10. A method according to Claim 9, **characterized in that** the valve opening time is chosen between 0.1 and 0.8 seconds.

11. A method according to any one of the preceding Claims, **characterized in that** with the progressive decrease in the milk flow as the end of milking approaches, the valve opening time is progressively somewhat increased or decreased for the fine calibration of the fat content contained in the analysis sample.

12. A method according to any one of Claims 1 to 11, **characterized in that** during every closing of the valve a part of the milk which has flowed through the valve is pumped back.

13. A method according to any one of Claims 1 to 12, **characterized in that** with every opening of the valve a suction force is exerted on the milk accelerating the start of milk flow.

14. A device for extracting an analysis sample representative of the total amount of milk milked from a cow, comprising a milk flow measuring device (6) disposed in a milking duct (4) and a processor unit (9) for controlling a milk sample extracting device (7) connected to an analysis sample container (8) and communicating with the milking duct, **characterized in that** the milk sample extracting device (7) comprises an electrically controllable magnetic coil (369; 413; 513) with which a sealing body (364; 407, 507) is moveable into a first position blocking a through flow opening (351; 405; 505) for the sample separating flow and to a second position releasing the opening, that the sealing body is moveable back and forth between the first and second positions at a controllable cycle time, and that the cycle time and the valve opening time in which the sealing body is in the second position releasing the through flow opening (351; 405; 505) can respectively be chosen in response to the total amount of milk to be expected, in such a manner that they lie within predetermined ranges of values, and wherein the valve opening time or the cycle time is controllable in response to the respectively measured milk flow via the processor unit, and that the valve opening time and the cycle time are varied in the same ratios to values lying within the ranges of values so as to avoid values of the valve opening time or the cycle time that lie outside the ranges of values due to a change in the milk flow.

15. A device according to Claim 14, **characterized in that** the sealing body (364; 407; 507) is made out of a permanent magnet or a ferromagnetic material and that a body (366; 403; 503) made out of a ferromagnetic material or a permanent magnet for stopping the sealing body in its first position, is disposed near to the through flow opening (351; 405; 505).

16. Device according to Claim 14 or 15, **characterized in that** the sealing body (407) is made out of a cylindrical body which is guided in lateral guide elements (408; 640, 641, 642).

17. Device according to Claim 15 or 16, **characterized in that** the ferromagnetic body or the body made out of a permanent magnet forms a pipe (403, 503) which forms the end of the through flow opening for the sample separating stream.

18. Device according to any one of Claims 15 to 17, **characterized in that** the sealing body (407; 507) and/or the end of the through flow opening (405; 505) facing the sealing body, is provided with a layer of absorbing or damping material (404; 504).

19. Device according to any one of Claims 14 to 18, **characterized in that** the magnetic coil (369; 613) is provided at the height of the second position of the sealing body (364; 607).

20. Device according to any one of Claims 14 to 19, **characterized in that** the magnetic coil (369; 413; 513; 613) is operable in the impulse mode.

21. Device according to any one of Claims 14 to 20, **characterized in that** for moving the sealing body from the first to the second position and vice-versa, the magnetic coil is controllable respectively with the help of impulses of different current direction.

22. Device according to Claim 21, **characterized in that** the impulses have a length between 10 and 100 msec.

23. Device according to any one of Claims 14 to 22, **characterized in that** the end (518) of the milk sample extracting device (530) in contact with the analysis sample container is connected with the milking vacuum.

24. Device according to any one of Claims 14 to 23, **characterized in that** the end of the milk sample extracting device (430) facing the analysis sample container is formed as a pipe end (418) insertable in the perforated covering (419) of the analysis sample container, and that a circular space (422) which is connected with the milking vacuum is formed between the outside of the pipe end (418) and a ring portion (420) surrounding said end, whose free end comes into contact with the upper surface of the covering (419).

25. Device according to Claim 23, **characterized in that** the end (519) of the milk sample extracting device is formed in such a way that the analysis sample container is attachable in a seal-proof manner onto this end.

26. Device according to any one of Claims 16 to 25, **characterized in that** the cylindrically shaped sealing body (407; 507; 607) is moveable along a substantially cylindrically shaped guideway in whose surfaces facing the sealing body, recesses (415, 416; 515; 615, 616, 643) are provided extending in the longitudinal direction of the sealing body, and which are in contact with the through flow opening (405; 505; 605) in the second position of the sealing body and are in contact with the end (418; 518) of the milk sample extracting device facing the analysis sample container, for conducting the milk through.

27. Device according to Claim 26, **characterized in that** the connection between the through flow opening (405; 505; 605) and the recesses (415, 416; 515; 615, 616, 643) is substantially interrupted with an adjusting movement of the sealing body (407; 507; 607) over a predetermined distance (D) before reaching the closing position of the through flow opening or respectively during the opening movement of the sealing body.

28. Device according to any one of the preceding claims, **characterized in that** the through flow opening (31) of the milk sample extracting device (30) opens into a chamber (22) in a direction opposing the direction of the sample separating flow and in which is set-up a milk build-up height (23) corresponding to the respective milk flow.

29. Device according to Claim 28, **characterized in that** devices (28) for measuring the milk build-up height are provided in the chamber (22).

30. Device according to any one of Claims 14 to 26, **characterized in that** the through flow opening (131) of the milk sample extracting device (130) opens into a chamber (125) in the direction opposing the direction of the sample separating flow and in which the milk milked from a cow is held respectively at a predetermined build-up height.

31. Device according to any one of Claims 14 to 26, **characterized in that** the through flow opening of the milk sample extracting device (230) is connected in the direction opposing the direction of the sample separating flow, with a sample extracting pipe (241, 341) which projects into a milk transporting duct (226).

32. Device according to Claim 31, **characterized in that** the sample extracting pipe (241; 341) has the longitudinal axis of its inlet opening so disposed that it is disposed from the inside wall of the milk transporting duct (226; 326) at around a distance of one third of the diameter.

33. Device according to any one of Claims 14 to 32, **characterized in that** a feedline for the milk is provided in the direction of the separating flow before the through flow opening, the feedline branching off into a first duct (352) which is connected with a discharging duct (363) in the analysis sample container (332) and surrounds the outlet opening, and into a second duct (351) connected with milk discharge flow, and that the sealing body (364) is moveable in such a way that, in its first position, it closes the outlet opening (352) and releases the second duct (351) and, in its second position, releases the outlet opening in the first duct (352) and closes the second duct (351).

## Revendications

1. Procédé de prélèvement d'un échantillon représentatif de la quantité totale de lait tirée d'une vache, procédé dans lequel des doses partielles d'échantillonnage quantitativement proportionnelles sont prélevées, au cours de l'opération d'extraction du lait, en fonction du débit de lait, c'est-à-dire de la quantité de lait extraite par unité de temps, caractérisé par le fait que, en utilisant une soupape commandée par cycles, chaque cycle englobant un temps d'ouverture de la soupape et un temps de fermeture de la soupape, un temps de cycle et un temps d'ouverture de la soupape sont déterminés en vue de prélever uniquement un échantillon maintenu en deçà d'une quantité maximale prédéterminée de 50 ml, sur la base de la valeur escomptée, acquise d'après l'expérience et relative à la quantité totale de lait devant être tirée de la vache considérée, le temps de cycle et le temps d'ouverture de la soupape étant respectivement choisis de manière qu'ils se situent à l'intérieur de plages de valeurs prédéterminées ; par le fait que le temps d'ouverture de la soupape ou le temps de cycle est commandé en fonction du débit de lait chaque fois mesuré ; et par le fait que, pour éviter des valeurs du temps d'ouverture de la soupape ou du temps de cycle situées en dehors des plages de valeurs, suite à la variation du débit de lait, le temps d'ouverture de la soupape et le temps de cycle sont modifiés dans des proportions identiques par rapport à des valeurs situées à l'intérieur des plages de valeurs.

2. Procédé selon la revendication 1, caractérisé par le fait que les doses partielles d'échantillonnage séparées sont maintenues sous la même pression que la quantité de lait venant d'être extraite.

3. Procédé selon la revendication 1 ou 2, caractérisé par le fait qu'une partie du lait, séparée d'avec le lait acheminé dans le conduit de transport et soumise à la pression cinétique du lait acheminé, est amenée à la soupape.

4. Procédé selon la revendication 1 ou 2, caractérisé par le fait que, en vue d'engendrer un courant séparé traversant la soupape et pour l'essentiel indépendant du débit du lait, la hauteur d'accumulation de lait est maintenue constante au-dessus de la soupape.

5. Procédé selon la revendication 1 ou 2, caractérisé par le fait que, en vue de modifier le courant séparé traversant la soupape, en fonction du débit du lait, une hauteur d'accumulation de lait chaque fois tributaire du débit du lait est engendrée au-dessus de la soupape.

6. Procédé selon la revendication 5, caractérisé par le fait que la plage de valeurs pour le temps d'ouverture de la soupape est modifiée, à chaque fois, en fonction de la hauteur mesurée d'accumulation de lait.

**7.** Procédé selon l'une des revendications 1 à 6, caractérisé par le fait que la plage de valeurs pour le temps de cycle est choisie entre 0,5 et 30 secondes (120 à 2 cycles par minute).

**8.** Procédé selon la revendication 7, caractérisé par le fait que la plage de valeurs pour le temps de cycle est choisie entre 2 et 30 secondes (30 à 2 cycles par minute).

**9.** Procédé selon l'une des revendications 1 à 8, caractérisé par le fait que la plage de valeurs pour le temps d'ouverture de la soupape est choisie entre 0,05 et 2 secondes.

**10.** Procédé selon la revendication 9, caractérisé par le fait que le temps d'ouverture de la soupape est choisi entre 0,1 et 0,8 seconde.

**11.** Procédé selon l'une des revendications précédentes, caractérisé par le fait que le temps d'ouverture de la soupape est quelque peu augmenté ou diminué de façon croissante au fur et à mesure de l'amenuisement du débit du lait vers la fin de la traite, en vue du calibrage fin de la teneur en graisse renfermée par l'échantillon.

**12.** Procédé selon l'une des revendications 1 à 11, caractérisé par le fait que, au cours de chaque fermeture de la soupape, une partie du lait ayant traversé la soupape est recyclée par pompage.

**13.** Procédé selon l'une des revendications 1 à 12, caractérisé par le fait que, lors de chaque ouverture de la soupape, le lait est soumis à une force d'aspiration accélérant l'amorce de l'écoulement du lait.

**14.** Dispositif de prélèvement d'un échantillon représentatif de la quantité totale de lait tirée d'une vache, comprenant un appareil de mesure (6) de débits de lait logé dans un conduit de traite (4), ainsi qu'une unité de traitement (9) pour commander un dispositif (7) de prélèvement d'échantillons de lait, relié à un réceptacle (8) d'échantillons et en communication avec le conduit de traite, caractérisé par le fait que le dispositif (7) de prélèvement d'échantillons de lait inclut une bobine magnétique (369 ; 413 ; 513) commandable électriquement, par laquelle un corps obturateur (364 ; 407 ; 507) peut être déplacé à une première position obturant un orifice (351 405 ; 505) d'écoulement du courant d'échantillonnage séparé, et à une seconde position dégageant cet orifice ; par le fait que le corps obturateur peut être animé d'un mouvement alternatif entre les première et seconde positions, avec un temps de cycle commandable ; par le fait que le temps de cycle et le temps d'ouverture de la soupape, durant lequel le corps obturateur occupe la seconde position dégageant l'orifice d'écoulement (351 ; 405 ; 505), peuvent être respectivement choisis, en fonction de la quantité totale de lait escomptée, de telle sorte qu'ils se trouvent à l'intérieur de plages de valeurs prédéterminées, le temps d'ouverture de la soupape ou le temps de cycle étant commandable, par l'intermédiaire de l'unité de traitement, en fonction du débit de lait chaque fois mesuré ; et par le fait que, pour éviter des valeurs du temps d'ouverture de la soupape ou du temps de cycle situées en dehors des plages de valeurs, suite à la variation du débit de lait, le temps d'ouverture de la soupape et le temps de cycle sont modifiés dans des proportions identiques par rapport à des valeurs situées à l'intérieur des plages de valeurs.

**15.** Dispositif selon la revendication 14, caractérisé par le fait que le corps obturateur (364 ; 407 ; 507) est respectivement constitué d'un aimant permanent ou d'un matériau ferromagnétique ; et par le fait qu'un corps (366 ; 403 ; 503) en un matériau ferromagnétique ou respectivement constitué d'un aimant permanent, maintenant le corps obturateur dans sa première position, est placé à proximité de l'orifice d'écoulement (351 ; 405 ; 505).

**16.** Dispositif selon la revendication 14 ou 15, caractérisé par le fait que le corps obturateur (407) est constitué d'un corps cylindrique guidé dans des guides latéraux (408 ; 640, 641, 642).

**17.** Dispositif selon la revendication 15 ou 16, caractérisé par le fait que le corps ferromagnétique ou, respectivement, le corps constitué d'un aimant permanent forme un tube (403, 503) matérialisant l'extrémité de l'orifice d'écoulement du courant d'échantillonnage séparé.

**18.** Dispositif selon l'une des revendications 15 à 17, caractérisé par le fait que le corps obturateur (407 ; 507), et/ou l'extrémité de l'orifice d'écoulement (405 ; 505) tournée vers ledit corps obturateur, est pourvu(e) d'une couche en un matériau d'amortissement (404 ; 504).

**19.** Dispositif selon l'une des revendications 14 à 18, caractérisé par le fait que la bobine magnétique (369 ; 613) est prévue à la hauteur de la seconde position du corps obturateur (364 ; 607).

**20.** Dispositif selon l'une des revendications 14 à 19, caractérisé par le fait que la bobine magnétique (369 ; 413 ; 513 ; 613) peut être actionnée en mode impulsionnel.

**21.** Dispositif selon l'une des revendications 14 à 20, caractérisé par le fait que la bobine magnétique peut être chaque fois commandée à l'aide d'impulsions à circulation de courant différente, afin de déplacer le corps obturateur de la première à la seconde position, et inversement.

**22.** Dispositif selon la revendication 21, caractérisé par le fait que les impulsions présentent une longueur comprise entre 10 et 100 msec.

**23.** Dispositif selon l'une des revendications 14 à 22, caractérisé par le fait que l'extrémité (518) du dispositif (530) de prélèvement d'échantillons de lait, reliée au réceptacle d'échantillons, est en communication avec la dépression de traite.

**24.** Dispositif selon l'une des revendications 14 à 23, caractérisé par le fait que l'extrémité du dispositif (430) de prélèvement d'échantillons de lait, tournée vers le réceptacle d'échantillons, est réalisée sous la forme d'une extrémité tubulaire (418) pouvant être introduite dans la coiffe perforée (419) du réceptacle d'échantillons ; et par le fait qu'une chambre annulaire (422), communiquant avec la dépression de traite, est formée entre la face extérieure de l'extrémité tubulaire (418) et une pièce annulaire (420) qui entoure cette extrémité, et dont l'extrémité libre vient s'appliquer contre la face supérieure de la coiffe (419).

**25.** Dispositif selon la revendication 23, caractérisé par le fait que l'extrémité (519) du dispositif de prélèvement d'échantillons de lait est réalisée de façon que le réceptacle d'échantillons puisse être emboîté de manière étanche sur cette extrémité.

**26.** Dispositif selon l'une des revendications 16 à 25, caractérisé par le fait que le corps obturateur cylindrique (407 ; 507 ; 607) est mobile le long d'une piste de guidage pour l'essentiel cylindrique, dont la surface tournée vers le corps obturateur présente des évidements (415, 416 ; 515 ; 615, 616, 643) qui s'étendent dans la direction longitudinale dudit corps obturateur et qui, dans la seconde position de ce corps obturateur, sont en communication avec l'orifice d'écoulement (405 ; 505 ; 605) et avec l'extrémité (418 ; 518) du dispositif de prélèvement d'échantillons de lait qui est tournée vers le réceptacle d'échantillons, en vue d'une circulation traversante de lait.

**27.** Dispositif selon la revendication 26, caractérisé par le fait que la communication, entre l'orifice d'écoulement (405 ; 505 ; 605) et les évidements (415, 416 ; 515 ; 615, 616, 643), est pour l'essentiel interrompue lors d'un mouvement de réglage du corps obturateur (407 ; 507 ; 607) sur un trajet prédéterminé (D) avant que la position d'obturation de l'orifice d'écoulement soit atteinte ou, respectivement, au cours du mouvement d'ouverture du corps obturateur.

**28.** Dispositif selon l'une des revendications précédentes, caractérisé par le fait que l'orifice d'écoulement (31) du dispositif (30) de prélèvement d'échantillons de lait débouche, dans une direction opposée à la direction du courant d'échantillonnage séparé, dans une chambre (22) dans laquelle s'instaure une hauteur (23) d'accumulation de lait correspondant au débit du lait considéré.

**29.** Dispositif selon la revendication 28, caractérisé par le fait que des dispositifs (28) sont prévus, dans la chambre (22), pour mesurer la hauteur d'accumulation de lait.

**30.** Dispositif selon l'une des revendications 14 à 26, caractérisé par le fait que l'orifice d'écoulement (131) du dispositif (130) de prélèvement d'échantillons de lait débouche, dans la direction opposée à la direction du courant d'échantillonnage séparé, dans une chambre (125) dans laquelle le lait soutiré est chaque fois maintenu à une hauteur d'accumulation prédéterminée.

**31.** Dispositif selon l'une des revendications 14 à 26, caractérisé par le fait que l'orifice d'écoulement du dispositif (230) de prélèvement d'échantillons de lait est relié, dans la direction opposée à la direction du courant d'échantillonnage séparé, à un tube (241, 341) de prélèvement d'échantillons qui fait intérieurement saillie dans un conduit (226) de transport de lait.

**32.** Dispositif selon la revendication 31, caractérisé par le fait que le tube (241 ; 341) de prélèvement d'échantillons occupe, avec l'axe longitudinal de son orifice d'admission, une position telle que ce dernier se trouve, par rapport à la paroi intérieure, à une distance représentant environ 1/3 du diamètre du conduit (226 ; 326) de transport de lait.

33. Dispositif selon l'une des revendications 14 à 32, caractérisé par le fait qu'un conduit d'arrivée du lait, prévu avant l'orifice d'écoulement dans la direction du courant séparé, se ramifie en un premier conduit (352) englobant l'orifice d'écoulement et relié à un conduit d'évacuation (363) gagnant le réceptacle (332) d'échantillons, et en un second conduit (351) raccordé à l'évacuation de lait ; et par le fait que le corps obturateur (364) est mobile de façon telle que, dans sa première position, il obture l'orifice d'écoulement (352) et dégage le second conduit (351) et que, dans sa seconde position, il dégage l'orifice d'écoulement pratiqué dans le premier conduit (352), et obture le second conduit (351).

FIG.1

FIG. 2

EP 0 643 292 B1

FIG.3

26

FIG.4

EP 0 643 292 B1

FIG.5

EP 0 643 292 B1

FIG.6

FIG.7

605  603  602  604

606

634

644

613  607  613

623  615

## FIG.8

641  616  640

644
623
643

607

642  615

## FIG.9

FIG.10

EP 0 643 292 B1